(19)

(11) **EP 2 794 539 B1**

(12) **EUROPÄISCHE PATENTSCHRIFT**

(45) Veröffentlichungstag und Bekanntmachung des Hinweises auf die Patenterteilung: **27.05.2015 Patentblatt 2015/22**

(21) Anmeldenummer: **12809717.7**

(22) Anmeldetag: **14.12.2012**

(51) Int Cl.:
***C07C 51/02*** (2006.01) ***C07C 51/09*** (2006.01)
***C07C 51/15*** (2006.01) ***C07C 51/44*** (2006.01)
***C07C 53/02*** (2006.01) ***C07C 53/06*** (2006.01)
***C07C 51/41*** (2006.01) ***C07C 51/48*** (2006.01)

(86) Internationale Anmeldenummer: **PCT/EP2012/075493**

(87) Internationale Veröffentlichungsnummer: **WO 2013/092403 (27.06.2013 Gazette 2013/26)**

(54) **VERFAHREN ZUR HERSTELLUNG VON AMEISENSÄURE**

METHOD FOR PRODUCING FORMIC ACID

PROCÉDÉ DE PRÉPARATION D'ACIDE FORMIQUE

(84) Benannte Vertragsstaaten: **AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.12.2011 EP 11194619**

(43) Veröffentlichungstag der Anmeldung: **29.10.2014 Patentblatt 2014/44**

(73) Patentinhaber: **BASF SE 67056 Ludwigshafen (DE)**

(72) Erfinder:
- **SCHNEIDER, Daniel 68159 Mannheim (DE)**
- **MOHL, Klaus-Dieter 68766 Hockenheim (DE)**
- **SCHÄFER, Martin 67269 Grünstadt (DE)**
- **FRIES, Donata, Maria 68199 Mannheim (DE)**
- **TELES, Joaquim, Henrique 67165 Waldsee (DE)**
- **BASSLER, Peter 68519 Viernheim (DE)**
- **RITTINGER, Stefan 68199 Mannheim (DE)**
- **SCHRAUB, Thomas 67433 Neustadt (DE)**

(56) Entgegenhaltungen:
**EP-A1- 0 001 432** **EP-A2- 0 563 831**
**WO-A2-2010/149507** **DE-B- 1 233 398**

Bemerkungen:
Die Akte enthält technische Angaben, die nach dem Eingang der Anmeldung eingereicht wurden und die nicht in dieser Patentschrift enthalten sind.

**EP 2 794 539 B1**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Anmeldung schließt durch Verweis die am 20. Dezember 2011 eingereichte vorläufige US-Anmeldung Nr. 61/577,703 ein.

Die vorliegende Erfindung betrifft ein Verfahren zur Gewinnung von Ameisensäure durch thermische Trennung eines Stroms enthaltend Ameisensäure und ein tertiäres Amin (I), bei dem man durch Zusammenbringen von tertiärem Amin (I) und einer Ameisensäurequelle in Gegenwart von Wasser einen flüssigen Strom enthaltend Ameisensäure, tertiäres Amin (I) und Wasser in einem Molverhältnis von Ameisensäure zu tertiärem Amin (I) von 0,5 bis 5 erzeugt, Wasser und organische Abbauprodukte des tertiäres Amins (I) entfernt, und aus dem erhaltenen flüssigen Strom in einer Destillationsvorrichtung bei einer Sumpftemperatur von 100 bis 300°C und einem Druck von 30 bis 3000 hPa abs Ameisensäure destillativ entfernt.

Ameisensäure ist ein wichtiges und vielseitig einsetzbares Produkt. Sie wird beispielsweise zum Ansäuern bei der Herstellung von Futtermitteln, als Konservierungsmittel, als Desinfektionsmittel, als Hilfsstoff in der Textil- und Lederindustrie, in Form ihrer Salze zur Enteisung von Flugzeugen und Start- und Landebahnen sowie als Synthesebaustein in der chemischen Industrie verwendet.

Das zurzeit wohl gängigste Verfahren zur Herstellung von Ameisensäure ist die Hydrolyse von Methylformiat, welches beispielsweise aus Methanol und Kohlenmonoxid gewonnen werden kann. Die durch Hydrolyse erhaltene wässrige Ameisensäure wird dann nachfolgend aufkonzentriert, beispielsweise unter Einsatz eines Extraktionshilfsmittels wie etwa eines Dialkylformamids (DE 25 45 658 A1).

Daneben ist die Gewinnung von Ameisensäure auch durch thermische Spaltung von Verbindungen aus Ameisensäure und einer tertiären Stickstoffbase bekannt. Bei diesen Verbindungen handelt es sich im Allgemeinen um saure Ammoniumformiate tertiärer Stickstoffbasen, bei denen die Ameisensäure über die Stufe der klassischen Salzbildung hinaus mit den tertiären Stickstoffbasen zu stabilen, über Wasserstoffbrückenbindungen verbrückten Additionsverbindungen reagiert hat. Die Additionsverbindungen aus Ameisensäure und tertiären Stickstoffbasen können durch Zusammenbringen der tertiären Stickstoffbase und einer Ameisensäurequelle gebildet werden. So offenbart beispielsweise WO 2006/021,411 die Herstellung solcher Additionsverbindungen allgemein (i) durch direkte Umsetzung der tertiären Stickstoffbase mit Ameisensäure, (ii) durch übergangsmetallkatalysierte Hydrierung von Kohlendioxid zu Ameisensäure in Gegenwart der tertiären Stickstoffbase, (iii) durch Umsetzung von Methylformiat mit Wasser und anschließender Extraktion der gebildeten Ameisensäure mit der tertiären Stickstoffbase, sowie (iv) durch Umsetzung von Methylformiat mit Wasser in Gegenwart der tertiären Stickstoffbase.

Die allgemeinen Vorteile beim Einsatz von Additionsverbindungen aus Ameisensäure und tertiären Stickstoffbasen zur Gewinnung von Ameisensäure liegen darin, dass einerseits die Additionsverbindungen die Ameisensäure zunächst stark genug binden, um die Ameisensäure als freie Ameisensäure aus dem Medium, beispielsweise dem Reaktionsmedium, in dem die Ameisensäure erst durch chemische Synthese gebildet wird, oder beispielsweise aus einer verdünnten Ameisensäurelösung zu entziehen und die Ameisensäure in Form ihrer Additionsverbindungen dadurch leichter abtrennen zu können, andererseits aber schwach genug sind, um die Ameisensäure nachfolgend aus den Additionsverbindungen durch thermische Spaltung wieder herauszulösen, um sie aufkonzentriert und gereinigt in freier Form zu gewinnen.

EP 0 001 432 A offenbart ein Verfahren zur Gewinnung von Ameisensäure durch Hydrolyse von Methylformiat in Gegenwart eines tertiären Amins, insbesondere eines Alkylimidazols, unter Bildung von Additionsverbindungen aus Ameisensäure und dem tertiären Amin. Das erhaltene Hydrolyse-Gemisch, welches nicht umgesetztes Methylformiat, Wasser, Methanol, Additionsverbindungen und tertiäres Amin enthält, wird in einer ersten Destillationskolonne von den Leichtsiedern Methylformiat und Methanol befreit. In einer zweiten Kolonne wird das verbleibende Sumpfprodukt entwässert. Das entwässerte Sumpfprodukt der zweiten Kolonne, welches noch Additionsverbindungen und tertiäres Amin enthält, wird dann einer dritten Kolonne zugeführt und darin die Additionsverbindungen thermisch in Ameisensäure und tertiäres Amin gespalten. Die freigesetzte Ameisensäure wird als Kopfprodukt entfernt. Das tertiäre Amin sammelt sich im Sumpf und wird zur Hydrolyse rückgeführt.

DE 34 28 319 A offenbart ein Verfahren zur Gewinnung von Ameisensäure durch Hydrolyse von Methylformiat. Das erhaltene Hydrolyse-Gemisch, welches nicht umgesetztes Methylformiat, Wasser, Methanol und Ameisensäure enthält, wird in einer ersten Destillationskolonne von den Leichtsiedern Methylformiat und Methanol befreit. Die im Sumpf anfallende wässrige Ameisensäure wird anschließend mit einem höhersiedenden Amin, insbesondere einem längerkettigen, hydrophoben $C_6$- bis $C_{14}$-Trialkylamin, in Gegenwart eines zusätzlichen hydrophoben Lösungsmittels, insbesondere eines aliphatischen, cycloaliphatischen oder aromatischen Kohlenwasserstoffs, extrahiert und dabei zu einer wässrigen Additionsverbindung aus Ameisensäure und dem Amin umgesetzt. Diese wird in einer zweiten Destillationskolonne entwässert. Die im Sumpf anfallende, entwässerte Additionsverbindung wird nun gemäß der Lehre von DE 34 28 319 A dem obersten Boden einer Destillationskolonne (in Abb. 1 als "K4" bezeichnet) zugeführt und thermisch gespalten. Das hydrophobe Lösungsmittel findet sich sowohl im Kopf als auch im Sumpf der Kolonne. Der gasförmige Kopfstrom enthält neben dem hydrophoben Lösungsmittel vor allem die freigesetzte Ameisensäure. Dieser Strom wird

im Kondensator wieder verflüssigt. Dabei bilden sich zwei Phasen aus, nämlich eine polare Ameisensäurephase und eine hydrophobe Lösungsmittelphase. Die Ameisensäurephase wird als Produkt abgeführt und die Lösungsmittelphase als Rücklauf in die Kolonne zurückgefahren. Durch die Gegenwart des hydrophoben Lösungsmittels kann eine vollständige Spaltung des Addukts erreicht werden, welche gemäß der Lehre der DE-Offenlegungsschrift angeblich ohne Zersetzung von Ameisensäure erfolgt. Der (fast) ameisensäurefreie Sumpf enthält das hydrophobe Amin sowie das hydrophobe Lösungsmittel. Dieser wird zur Extraktionsstufe rückgeführt.

EP 0 181 078 A und EP 0 126 524 A beschreiben Verfahren zur Gewinnung von Ameisensäure durch Hydrierung von Kohlendioxid in Gegenwart eines Übergangsmetallkatalysators und eines tertiären Amins wie beispielsweise eines $C_1$- bis $C_{10}$-Trialkylamins unter Bildung einer Additionsverbindung aus Ameisensäure und dem tertiären Amin, Aufarbeitung des Hydrieraustrags unter Abtrennung des Katalysators und der Leichtsieder, Austausch der Aminbase durch ein schwächeres, höher siedendes tertiäres Amin, insbesondere durch ein Alkylimidazol, unter Abtrennung des ersten tertiären Amins und nachfolgender thermischer Spaltung der neu gebildeten Additionsverbindung in einer Destillationskolonne. Dazu wird gemäß EP 0 181 078 A, Fig. 1, der Ameisensäure und Amin enthaltende Strom in den mittleren Bereich der Kolonne "30" zugeführt. Die bei der thermischen Spaltung freigesetzte Ameisensäure wird als Kopfprodukt entfernt. Das schwächere, höher siedende tertiäre Amin sammelt sich im Sumpf und wird zur Stufe des Basenaustauschs rückgeführt.

WO 2008/116,799 offenbart ein Verfahren zur Gewinnung von Ameisensäure durch Hydrierung von Kohlendioxid in Gegenwart eines Übergangsmetallkatalysators, eines hochsiedenden polaren Lösungsmittels wie beispielsweise eines Alkohols, Ethers, Sulfolans, Dimethylsulfoxids oder Amids, und eines, mindestens eine Hydroxylgruppe tragenden, polaren Amins unter Bildung einer Additionsverbindung aus Ameisensäure und dem Amin. Gemäß der Lehre von WO 2008/116,799 kann der Hydrieraustrag zur thermischen Spaltung der Additionsverbindung direkt einer Destillationsvorrichtung zugeführt werden. Diese kann eine Destillationskolonne und, falls kurze Verweilzeiten gewünscht sind, auch einen Dünnschicht- oder Fallfilm-Verdampfer umfassen. Die freigesetzte Ameisensäure wird als Kopfprodukt entfernt. Das polare Amin und das polare Lösungsmittel sowie gegebenenfalls nicht abgetrennter Katalysator sammeln sich im Sumpf und können zur Hydrierstufe rückgeführt werden.

WO 2006/021,411 beschreibt ein Verfahren zur Gewinnung von Ameisensäure durch thermische Spaltung einer Additionsverbindung aus Ameisensäure und einem tertiären Amin (quartäres Ammoniumformiat), bei dem das tertiäre Amin einen Siedepunkt von 105 bis 175°C aufweist. Als bevorzugte tertiäre Amine sind Alkylpyridine genannt. Durch den speziellen Siedebereich der tertiären Amine wird die Farbstabilität der gewonnenen Ameisensäure erhöht. Die einzusetzende Additionsverbindung kann dabei allgemein aus dem tertiären Amin und einer Ameisensäurequelle gewonnen werden. Vorteilhafterweise wird der Austrag aus der Adduktsynthese zunächst von flüchtigen Bestandteilen befreit und dann der thermischen Spaltung zugeführt. Die thermische Spaltung erfolgt wie gewohnt in einer Destillationskolonne, wobei der Ameisensäure und Amin enthaltende Strom gemäß Fig. 1 von WO 2006/021,411 in den mittleren Bereich der Kolonne (C) zugeführt wird. Die freigesetzte Ameisensäure wird als Kopfprodukt entfernt. Das tertiäre Amin, welches gegebenenfalls noch Reste an Ameisensäure enthalten kann, sammelt sich im Sumpf und kann zur Ameisensäurequelle rückgeführt werden.

EP 0 563 831 A nennt ein verbessertes Verfahren zur thermischen Spaltung einer Additionsverbindung aus Ameisensäure und einem tertiären Amin (quartäres Ammoniumformiat) unter Gewinnung von Ameisensäure. Die einzusetzende Additionsverbindung kann dabei allgemein aus dem tertiären Amin und einer Ameisensäurequelle gewonnen werden. Vorteilhafterweise wird der Austrag aus der Synthese zunächst von flüchtigen Bestandteilen befreit und dann zur thermischen Spaltung in die Mitte einer Destillationskolonne zugeführt. Die Verbesserung besteht im Wesentlichen darin, die thermische Spaltung der Additionsverbindung in Gegenwart eines sekundären Formamids durchzuführen, welches die Farbstabilität der gewonnenen Ameisensäure erhöht. Die freigesetzte Ameisensäure wird als Kopfprodukt entfernt. Das tertiäre Amin sowie das sekundäre Formamid sammeln sich im Sumpf und können zur Ameisensäurequelle rückgeführt werden.

PCT/EP2011/060770 lehrt ein Verfahren zur Gewinnung von Ameisensäure durch thermische Trennung eines Stroms enthaltend Ameisensäure und ein tertiäres Amin (I), bei dem man durch Zusammenbringen von tertiärem Amin (I) und einer Ameisensäurequelle einen flüssigen Strom enthaltend Ameisensäure und ein tertiäres Amin (I) in einem Molverhältnis von 0,5 bis 5 erzeugt, 10 bis 100 Gew.-% der darin enthaltenen Nebenkomponenten abtrennt, und aus dem erhaltenen flüssigen Strom in einer Destillationsvorrichtung bei einer Sumpftemperatur von 100 bis 300°C und einem Druck von 30 bis 3000 hPa abs Ameisensäure destillativ entfernt, wobei man den Sumpfaustrag aus der Destillationsvorrichtung in zwei flüssige Phasen trennt, von denen die obere Flüssighase an tertiärem Amin (I) angereichert ist und zur Ameisensäurequelle rückgeführt wird und die untere Flüssighase an Ameisensäure angereichert ist und zur Abtrennung der Nebenkomponenten und/oder zur Destillationsvorrichtung rückgeführt wird.

Aufgabe der vorliegenden Erfindung war es, ein verbessertes Verfahren zur Gewinnung von Ameisensäure durch thermische Trennung eines Stroms enthaltend Ameisensäure und ein tertiäres Amin zu finden, welches Vorteile gegenüber dem Stand der Technik aufweist und welches Ameisensäure in hoher Ausbeute und hoher Konzentration zu gewinnen vermag. Insbesondere soll das verbesserte Verfahren auch über längere Betriebszeiten hinweg stabil

funktionieren und Ameisensäure in konstant hoher Reinheit produzieren. Natürlich soll das Verfahren möglichst einfach und auch möglichst energiegünstig durchgeführt werden können.

Überraschend wurde ein Verfahren zur Gewinnung von Ameisensäure durch thermische Trennung eines Stroms enthaltend Ameisensäure und ein tertiäres Amin (I), welches bei einem Druck von 1013 hPa abs einen um mindestens 5°C höheren Siedepunkt als Ameisensäure aufweist und welches die allgemeine Formel (I)

$$NR^1R^2R^3 \qquad (I)$$

aufweist, in der die Reste $R^1$ bis $R^3$ unabhängig voneinander ausgewählt sind aus der Gruppe $C_5$- bis $C_8$-Alkyl, gefunden, bei dem man

(a) durch Zusammenbringen von tertiärem Amin (I) und einer Ameisensäurequelle in Gegenwart von Wasser einen flüssigen Strom enthaltend Ameisensäure, tertiäres Amin (I) und Wasser erzeugt, wobei

(i) man eine Ameisensäurequelle einsetzt, welche Methylformiat enthält und aus welcher der flüssige Strom enthaltend Ameisensäure, tertiäres Amin (I) und Wasser durch Hydrolyse von Methylformiat erhalten wird; oder

(ii) man eine Ameisensäurequelle einsetzt, welche Kohlendioxid enthält und aus welcher der flüssige Strom enthaltend Ameisensäure, tertiäres Amin (I) und Wasser durch übergangsmetallkatalysierte Hydrierung von Kohlendioxid erhalten wird,
und der flüssige Strom enthaltend Ameisensäure, tertiäres Amin (I) und Wasser ein Molverhältnis von Ameisensäure zu tertiärem Amin (I) von 0,5 bis 5 aufweist;

(b) aus dem aus Schritt (a) erhaltenen flüssigen Strom Wasser und organische Abbauprodukte des tertiären Amins (I) abtrennt, wobei die organischen Abbauprodukte des tertiären Amins (I) bereits in dem Schritt (a) zugeführten tertiären Amin (I) enthalten waren und/oder im Laufe des Verfahrens bis zum vorliegenden Schritt (b) entstanden, und einen an Wasser und organischen Abbauprodukten des tertiären Amins (I) abgereicherten flüssigen Strom enthaltend Ameisensäure und tertiäres Amin (I) erhält; und

(c) aus dem aus Schritt (b) erhaltenen flüssigen Strom enthaltend Ameisensäure und tertiäres Amin (I) in einer Destillationsvorrichtung bei einer Sumpftemperatur von 100 bis 300°C und einem Druck von 30 bis 3000 hPa abs Ameisensäure destillativ entfernt;
und welches dadurch gekennzeichnet ist, dass man

(b1) den in Schritt (b) abgetrennten Strom enthaltend Wasser und organische Abbauprodukte des tertiären Amins (I) in zwei flüssige Phasen trennt;

(b2) die obere, an organischen Abbauprodukten des tertiärem Amins (I) angereicherte Flüssighase entfernt; und

(b3) die untere, Wasser enthaltende Flüssigphase zu Schritt (a) rückführt.

Das beim erfindungsgemäßen Verfahren in Schritt (a) einzusetzende tertiäre Amin (I) weist bei einem Druck von 1013 hPa abs einen um mindestens 5°C höheren Siedepunkt als Ameisensäure auf. Bevorzugt weist das einzusetzende tertiäre Amin (I) einen um mindestens 10°C, besonders bevorzugt einem um mindestens 50°C und ganz besonders bevorzugt einem um mindestens 100°C höheren Siedepunkt als Ameisensäure auf. Eine Einschränkung hinsichtlich eines oberen Grenzwertes für den Siedepunkt ist nicht erforderlich, da für das erfindungsgemäße Verfahren ein möglichst niedriger Dampfdruck des tertiären Amin (I) grundsätzlich von Vorteil ist. Im Allgemeinen liegt der Siedepunkt des tertiären Amins (I) bei einem, gegebenenfalls nach bekannten Methoden vom Vakuum auf 1013 hPa abs hochgerechneten Drucks, unterhalb von 500°C.

Von besonderer technischer Bedeutung sind zum Zeitpunkt dieser Patentanmeldung die Erzeugung der Ameisensäure durch Hydrolyse von Methylformiat sowie die Erzeugung der Ameisensäure durch übergangsmetallkatalysierte Hydrierung von Kohlendioxid. Beide genannten Synthesemöglichkeiten sind in der Fachwelt wohlbekannt und in verschiedenen Varianten und Ausführungsformen vorbeschrieben.

Im Falle der Hydrolyse von Methylformiat werden üblicherweise Methylformiat, Wasser und tertiäres Amin (I) gemeinsam oder nacheinander in den Hydrolysereaktor gegeben, um die durch die Hydrolyse gebildete Ameisensäure mit dem tertiären Amin (I) in Form einer Additionsverbindung abzufangen und so dem Hydrolysegleichgewicht zu entziehen. Dadurch ist ein hoher Umsatz an Methylformiat erreichbar und eine besonders vorteilhafte Abtrennung des nicht umgesetzten Wassers durch eine nachfolgende Destillation möglich.

Im Falle der übergangsmetallkatalysierten Hydrierung von Kohlendioxid wird das tertiäre Amin (I) im Allgemeinen in den Hydrierreaktor gegeben, um bereits bei der Hydrierung eine Ameisensäure und ein tertiäres Amin (I) enthaltenden Strom zu bilden. Bevorzugt ist in Schritt (a) die Erzeugung des Stroms enthaltend Ameisensäure und tertiäres Amin (I) durch Hydrolyse von Methylformiat in Gegenwart von Wasser und tertiärem Amin (I).

Der Gehalt an Wasser beim Zusammenbringen des tertiären Amins (I) und der Ameisensäurequelle in Schritt (a) ist so einzustellen, dass der in Schritt (a) erzeugte flüssige Strom neben Ameisensäure und tertiärem Amin (I) auch Wasser enthält. So wird beispielsweise bei der Darstellung von Ameisensäure durch Hydrolyse von Methylformiat jeweils Wasser zur Darstellung der Ameisensäure chemisch verbraucht. Entsprechend ist dann bei der Zugabe des Wassers die Menge an chemisch verbrauchtem Wasser mit zu berücksichtigen.

Das Zusammenbringen von tertiärem Amin (I) und der Ameisensäurequelle kann auf die unterschiedlichste Art und Weise erfolgen.Es ist im Allgemeinen vorteilhaft, die Ameisensäurequelle erst durch Zusammenführen der einzelnen Komponenten im Reaktor zu erzeugen. Als Reaktoren kommen dabei insbesondere die dem Fachmann für die Art der Reaktion bekannten Reaktoren in Frage. Das tertiäre Amin (I) kann dabei beispielsweise bereits vorgelegt sein, parallel zu den einzelnen Komponenten der Ameisensäurequelle zugeführt werden, im Verlaufe der chemischen Reaktion zugeführt werden oder erst am Ende der chemischen Reaktion zugeführt werden. Es ist auch möglich, diese einzelnen Schritte auf mehrere Reaktoren zu verteilen. Je nach Exothermie des Zusammenbringens von tertiärem Amin (I) und der Ameisensäurequelle mag es vorteilhaft sein, die Vorrichtung selbst oder den daraus erhaltenen Strom zu kühlen.

Geeignete Arbeitsweisen zum Zusammenbringen von tertiärem Amin (I) und der Ameisensäurequelle sind mit dem üblichen Fachwissen ohne großen Aufwand ermittelbar.

Der beim Zusammenbringen von tertiärem Amin (I) und einer Ameisensäurequelle in Schritt (a) erzeugte flüssige Strom weist ein Molverhältnis von Ameisensäure zu tertiärem Amin (I) von 0,5 bis 5 auf. Das Molverhältnis liegt bevorzugt bei $\geq 1$ sowie bevorzugt bei $\leq 3$. Das genannte Molverhältnis bezieht sich auf den gesamten Flüssigkeitsstrom unabhängig davon, ob dieser ein- oder mehrphasig vorliegt.

Der in Schritt (a) erzeugte flüssige Strom enthaltend Ameisensäure, tertiäres Amin (I) und Wasser weist im Allgemeinen eine Konzentration an Ameisensäure plus tertiärem Amin (I) von 1 bis 99 Gew.-% bezogen auf die Gesamtmenge des Stroms auf. Bevorzugt weist der genannte Strom eine Konzentration an Ameisensäure plus tertiärem Amin (I) von $\geq 5$ Gew.-% und besonders bevorzugt von $\geq 15$ Gew.-% sowie bevorzugt von $\leq 95$ Gew.-% und besonders bevorzugt von $\leq 90$ Gew.-% auf. Der auf 100 Gew.-% verbleibende Anteil des Stroms setzt sich zusammen aus Wasser, gegebenenfalls organischen Abbauprodukten des tertiären Amins (I) und Nebenkomponenten wie im weiteren Verlauf der vorliegenden Beschreibung noch definiert. Bezogen auf diesen verbleibenden Anteil beträgt der gewichtsmäßige Anteil an Wasser im Allgemeinen 1 bis $\leq 100\%$, bevorzugt $\geq 5\%$ und besonders bevorzugt $\geq 10\%$ sowie bevorzugt $\leq 99\%$ und besonders bevorzugt $\leq 95\%$.

Aus dem aus Schritt (a) erhaltenen flüssigen, und neben Ameisensäure und tertiärem Amin (I) auch Wasser enthaltenden Strom ist es zur Aufkonzentrierung der Ameisensäure angebracht, Wasser abzutrennen. Überraschend wurde dabei im Rahmen der vorliegenden Erfindung gefunden, dass es in diesem Schritt möglich und auch besonders vorteilhaft ist, neben dem Wasser auch organische Abbauprodukte des tertiären Amins (I) abzutrennen. Daher werden beim erfindungsgemäßen Verfahren in Schritt (b) aus dem in Schritt (a) erhaltenen flüssigen Strom Wasser und organische Abbauprodukte des tertiären Amins (I) abgetrennt, wobei die organischen Abbauprodukte des tertiären Amins (I) bereits in dem Schritt (a) zugeführten tertiären Amin (I) enthalten waren und/oder im Laufe des Verfahrens bis zum vorliegenden Schritt (b) entstanden, und somit ein an Wasser und organischen Abbauprodukten des tertiären Amins (I) abgereicherter flüssiger Strom enthaltend Ameisensäure und tertiäres Amin (I) erhalten.

Unter dem Begriff organische Abbauprodukte des tertiären Amins (I) sind dabei Verbindungen zu verstehen, welche sich unter einer chemischen Umwandlung des tertiären Amins (I) unter Trennung ursprünglich vorhandener Bindungen, Neuknüpfung von Stickstoff-Kohlenstoff-Bindungen oder chemischer Umwandlung der am Stickstoff gebundenen Reste bilden. So wurde im Rahmen der Erfindung erkannt, dass tertiäre Amine (I) beispielsweise dazu neigen, sich in Gegenwart von Ameisensäure unter erhöhter Temperatur und erhöhtem Druck, wie sie in einzelnen Stufen des erfindungsgemäßen Verfahrens vorliegen, sich in das entsprechende, mit den Resten des tertiären Amins (I) N,N-substituierte Formamid und das entsprechende, den anderen Rest des tertiären Amins (I) enthaltende Formiat zu zersetzen. Im Falle eines tertiären Amins (I) mit drei identischen Resten R, wie beispielsweise $C_5$- bis $C_8$-Alkyl, würde die genannte Zersetzungsreaktion beispielsweise wie folgt lauten:

$$NR_3 + 2\,HC(=O)OH \xrightarrow{-H_2O} HC(=O)NR_2 + HC(=O)OR, \quad (A)$$

wobei sich hierbei das entsprechende Dialkylformamid und das entsprechende Alkylformiat als organische Abbauprodukte des tertiären Amins (I) bilden. Des Weiteren wurde im Rahmen der Erfindung erkannt, dass tertiäre Amine (I) beispielsweise auch dazu neigen, sich in Gegenwart von Ameisensäure und Spuren von Sauerstoff unter erhöhter Temperatur, wie sie in einzelnen Stufen des erfindungsgemäßen Verfahrens vorliegen können, sich in das entsprechende, mit den Resten des tertiären Amins (I) N,N-substituierte Formamid und den, aus dem den anderen Rest entstandenen Aldehyd zu zersetzen. Im Falle eines tertiären Amins (I) mit drei identischen Resten $CH_2$-R, wie beispielsweise $C_5$- bis $C_8$-Alkyl, würde die genannte Zersetzungsreaktion beispielsweise wie folgt lauten:

$$N(CH_2R)_3 + H{-}C(=O)OH \xrightarrow[-\ H_2O]{+\ ^1/_2\ O_2} H{-}C(=O){-}N(CH_2R)_2 + R{-}C(=O){-}H \quad , \quad (B)$$

wobei sich hierbei das entsprechende Dialkylformamid und das entsprechende Alkanal als organische Abbauprodukte des tertiären Amins (I) bilden.

Die beim erfindungsgemäßen Verfahren abzutrennenden organischen Abbauprodukte des tertiären Amins (I) sind entweder bereits in dem Schritt (a) zugeführten tertiären Amin (I) enthalten und/oder werden im Laufe des Verfahrens bis zum vorliegenden Schritt (b) erst gebildet. So ist es beispielsweise möglich, dass das in Schritt (a) zugeführte tertiäre Amin (I) aufgrund seiner Herstellung oder Vorbehandlung diverse organische Abbauprodukte des tertiären Amins (I) bereits enthält. Dies ist im Allgemeinen bei einer Rückführung des in Schritt (c) nach der destillativen Entfernung der Ameisensäure erhaltenen tertiären Amins (I) zu Schritt (a) der Fall. Es ist aber auch möglich, dass sich die abzutrennenden organischen Abbauprodukte des tertiären Amins (I) ausschließlich oder zusätzlich zu den bereits im tertiären Amin (I) zugeführten bei entsprechenden Bedingungen erst in Schritt (a) bilden und/oder erst bei der Abtrennung des Wassers in Schritt (b) und/oder in optionalen Zwischenschritten zwischen Schritt (a) und Schritt (b).

Die Menge der organischen Abbauprodukte des tertiären Amins (I), die beim erfindungsgemäßen Verfahren in Schritt (b) üblicherweise abgetrennt wird, kann je nach Durchführung des Verfahrens sehr unterschiedlich sein. Maßgebend hierfür sind vor allem die Reinheit des eingesetzten tertiären Amins (I), die Tatsache, ob das in Schritt (c) erhaltene tertiäre Amin (I) rückgeführt wird oder nicht, die weiteren Bedingungen im Verfahren (z.B. Temperatur, Druck, Anwesenheit von Sauerstoff, Konzentrationsverhältnisse, etc. in den einzelnen Verfahrensschritten). Im Allgemeinen wird beim erfindungsgemäßen Verfahren in Schritt (b) eine Menge an organischen Abbauprodukten des tertiären Amins (I) abgetrennt, welche 5 Gew.-ppm bis 5 Gew.-% der Menge des zu Schritt (b) zugeführten Gesamtstroms entspricht. Unter Gesamtstrom ist dabei der gesamte, zu Schritt (b) zugeführte Strom, also einschließlich Ameisensäure, tertiäres Amin (I), Wasser und gegebenenfalls weiterer Komponenten, zu verstehen. Bevorzugt werden $\geq$ 10 Gew.-ppm und besonders bevorzugt $\geq$ 30 Gew.-ppm, sowie ferner bevorzugt $\leq$ 2 Gew.-% und besonders bevorzugt $\leq$ 1 Gew.-% abgetrennt.

Die organischen Abbauprodukte des tertiären Amins (I) können sich ohne die erfindungsgemäße Abtrennung nachteilig auf das Verfahren auswirken. So können diese beispielsweise je nach Art und Konzentration die Reinheit der in Schritt (c) destillativ gewonnenen Ameisensäure negativ beeinflussen oder zur Sicherstellung einer hohen Reinheit beispielsweise in Schritt (c) eine aufwändigere Destillationsvorrichtung (Notwendigkeit einer größeren Anzahl an theoretischen Trennstufen) oder sogar eine separate Nachdestillation bedingen. Ferner können sich die organischen Abbauprodukte des tertiären Amins (I) im Falle einer Rückführung des in Schritt (c) nach der destillativen Entfernung der Ameisensäure erhaltenen tertiären Amins (I) zu Schritt (a) im Verfahren aufpegeln und den zuvor beschriebenen nachteiligen Effekt hinsichtlich der Reinheit der gewonnenen Ameisensäure signifikant verstärken. Zusätzlich wurde im Rahmen der vorliegenden Erfindung überraschend gefunden, dass bei einer weiteren, weiter unten im Text beschriebenen, besonders bevorzugten Variante, bei der der Sumpf aus der Destillationsvorrichtung des Schrittes (c) in zwei Phasen getrennt und separat an separate Stellen im Verfahren rückgeführt wird, die Qualität der Phasentrennung mit steigendem Gehalt an organischen Abbauprodukte des tertiären Amins (I) leidet und sich beide Phasen zunehmend mischen beziehungsweise im Extremfall eine Phasentrennung sogar gänzlich unterbleibt. Als Folge davon wird in den beiden Rückführströmen einer höherer Gehalt der darin unerwünschten Komponente transportiert, was zu einer allgemeinen Erhöhung der Stoffströme im Verfahren führt und somit letztendlich größere Apparate und Leitungen als auch einen höheren Energiebedarf für den Betrieb bedingt.

Weiterhin wurde im Rahmen der vorliegenden Erfindung überraschend gefunden, dass sich der in Schritt (b) abgetrennte Strom enthaltend Wasser und organische Abbauprodukte des tertiären Amins (I) in zwei flüssige Phasen trennt. Schritt (b1) der vorliegenden Erfindung umfasst daher die Trennung des in Schritt (b) abgetrennten Stroms enthaltend Wasser und organische Abbauprodukte des tertiären Amins (I) in zwei flüssige Phasen. Die obere, an organischen Abbauprodukten des tertiären Amins (I) angereicherte Flüssighase wird in Schritt (b2) entfernt und die untere, Wasser enthaltende Flüssigphase in Schritt (b3) zu Schritt (a) rückgeführt. Die Entfernung der oberen, an organischen

Abbauprodukten des tertiären Amins (I) angereicherten Flüssighase in Schritt (b2) kann dabei beispielsweise kontinuierlich oder diskontinuierlich erfolgen. Der Austrag an organischen Abbauprodukten des tertiären Amins (I) kann dann beispielsweise entsorgt werden, wobei hierbei auch eine thermische Verwertung in Frage kommt. Es ist aber gegebenenfalls auch möglich, den Austrag als Einsatz- oder Rohstoff für Synthesen einzusetzen.

Die Abtrennung von Wasser und organischen Abbauprodukten des tertiären Amins (I) in Schritt (b) aus dem in Schritt (a) erhaltenen flüssigen Strom erfolgt bevorzugt destillativ. Als Destillationsvorrichtungen hierfür kommen grundsätzlich die dem Fachmann für derartige Trennaufgaben bekannte, beziehungsweise mit seinem allgemeinen Fachkönnen auszulegende Vorrichtungen in Frage. Die Sumpftemperatur liegt vorteilhafterweiser im Bereich von 100 bis 300°C, bevorzugt 120 bis 290°C, besonders bevorzugt 150 bis 280°C, der Druck vorteilhafterweiser im Bereich von 100 bis 4000°hPa abs.

Aus dem in Schritt (a) erhaltenen flüssigen Strom können in Schritt (b) neben Wasser und organischen Abbauprodukten des tertiären Amins (I) natürlich auch weitere Komponenten, die im Folgenden vereinfacht Nebenprodukte genannt sind, abgetrennt werden. Unter dem Begriff Nebenkomponenten sind dabei alle in dem in Schritt (a) erhaltenen flüssigen Strom enthaltenen Komponenten zu verstehen, bei denen es sich nicht um Ameisensäure, tertiäres Amin (I), Wasser oder organische Abbauprodukte des tertiären Amins (I) handelt. Als Beispiele seien etwa genannt Methanol (insbesondere bei der Hydrolyse von Methylformiat), nicht hydrolysiertes Methylformiat (insbesondere bei der Hydrolyse von Methylformiat), gelöste Inertgase, homogener Katalysator (insbesondere bei der Hydrierung von Kohlendioxid), gelöstes Kohlendioxid beziehungsweise gelöster Wasserstoff (insbesondere bei der Hydrierung von Kohlendioxid), Lösungsmittel, sonstige Komponenten.

Die Art und Weise wie gegebenenfalls die Nebenkomponenten abgetrennt werden ist für das erfindungsgemäße Verfahren unerheblich. So können beispielsweise die üblichen und bekannten Methoden zur Trennung von flüssigen Stoffgemischen eingesetzt werden. Allem voran sei hier die destillative Trennung genannt. So können beispielsweise leichtsiedende Nebenkomponenten wie etwa Methanol oder Methylformiat über Kopf oder als Seitenabzug einer Destillationsvorrichtung abgetrennt werden. Es ist aber auch denkbar, schwersiedende Nebenkomponenten über Sumpf und das Ameisensäure und tertiäre Amin (I) enthaltende Gemisch als Seitenstrom oder Kopfprodukt abzutrennen. Neben der destillativen Trennung sind auch Membran-, Absorptions-, Adsorptions-, Kristallisations-, Filtrations-, Sedimentations- oder Extraktionsverfahren möglich.

Es können natürlich auch mehrere Trennschritte, die des Weiteren auch auf unterschiedliche Methoden beruhen können, kombiniert werden. Die Auslegung des Trennschrittes beziehungsweise der Trennschritte kann mit dem üblichen Fachwissen vorgenommen werden.

Die mögliche Abtrennung von Nebenprodukten in Schritt (b) kann dabei grundsätzlich vor oder nach der Abtrennung von Wasser und organischen Abbauprodukten des tertiären Amins (I) erfolgen. Maßgeblich für die Reihenfolge sind vorwiegend praktische Gesichtspunkte beziehungsweise beim Einsatz eines oder mehrerer destillativer Schritte die entsprechenden Stoffeigenschaften. Im Falle der destillativen Abtrennung von Methanol und/oder nicht umgesetzten Methylformiat beim Einsatz von Methylformiat als Ameisensäurequelle erfolgt deren Abtrennung aufgrund der Lage der Siedepunkte vor der beispielsweise ebenfalls destillativen Abtrennung von Wasser und organischen Abbauprodukten des tertiären Amins (I).

Die Menge an dem in Schritt (b) abgetrennten Wassers beträgt beim erfindungsgemäßen Verfahren im Allgemeinen 10 bis 100% der im Strom aus Schritt (a) enthaltenen Wassermenge. Bevorzugt werden in Schritt (b) $\geq$ 20% und besonders bevorzugt $\geq$ 30% sowie bevorzugt $\leq$ 97% und besonders bevorzugt $\leq$ 95% der im Strom aus Schritt (a) enthaltenen Wassermenge abgetrennt.

Zwischen den Schritten (a) und (c) können beim erfindungsgemäßen Verfahren neben Schritt (b) selbstverständlich noch weitere Verfahrensschritte durchgeführt werden.

Aus dem aus Schritt (b) erhaltenen flüssigen Strom wird schließlich in einer Destillationsvorrichtung bei einer Sumpftemperatur von 80 bis 300°C, bevorzugt 100 bis 300°C, und einem Druck von 30 bis 3000 hPa abs Ameisensäure destillativ entfernt. Als Destillationsvorrichtungen hierfür kommen grundsätzlich die dem Fachmann für derartige Trennaufgaben bekannte, beziehungsweise mit seinem allgemeinen Fachkönnen auszulegende Vorrichtungen in Frage.

Üblicherweise umfasst die Destillationsvorrichtung neben dem eigentlichen Kolonnenkörper mit Einbauten unter anderem einen Kopfkondensator und einen Sumpfverdampfer. Daneben kann diese natürlich auch noch weitere periphere Vorrichtungen oder interne Einbauten umfassen, wie beispielsweise einen Flashbehälter im Zulauf (beispielsweise zur Auftrennung von Gas und Flüssigkeit im Zulauf zum Kolonnenkörper), einen Zwischenverdampfer (beispielsweise zur verbesserten Wärmeintegration des Verfahrens) oder Einbauten zur Vermeidung beziehungsweise Reduzierung der Aerosolbildung (wie zum Beispiel temperierbare Böden, Demister, Koaleszer oder Tiefbettdiffusionsfilter). Der Kolonnenkörper kann beispielsweise mit Packungen, Füllkörpern oder Böden bestückt sein. Die Zahl der erforderlichen Trennstufen ist insbesondere abhängig von der Art des tertiären Amins (I), der Konzentration an Ameisensäure und tertiärem Amin (I) im Zulauf der Destillationsvorrichtung in Schritt (c) und der gewünschten Konzentration beziehungsweise dem gewünschten Reinheitsgrad der Ameisensäure, und ist vom Fachmann in üblicher Weise ermittelbar. Im Allgemeinen liegt die Zahl der erforderlichen Trennstufen bei $\geq$ 3, bevorzugt bei $\geq$ 6 und besonders bevorzugt bei $\geq$ 7.

Nach oben hin sind prinzipiell keine Grenzen gesetzt. Aus praktischen Gründen dürfte es aber üblich sein, in der Regel ≤ 70, gegebenenfalls ≤ 50 Trennstufen oder sogar ≤ 30 Trennstufen einzusetzen.

Der Ameisensäure und tertiäres Amin (I) enthaltende Strom aus Schritt (b) kann in der Destillationsvorrichtung beispielsweise als Seitenstrom dem Kolonnenkörper zugeführt werden.

Gegebenenfalls kann der Zugabe zum Beispiel auch ein Flashverdampfer vorgeschaltet sein. Um die thermische Belastung des zugeführten Stroms in der Destillationsvorrichtung so gering wie möglich zu halten, ist es im Allgemeinen von Vorteil, diesen eher dem unteren Bereich der Destillationsvorrichtung zuzuführen. So ist es bevorzugt, in Schritt (c) den Strom enthaltend Ameisensäure und ein tertiäres Amin (I) im Bereich des unteren Viertels, bevorzugt im Bereich des unteren Fünftels und besonders bevorzugt im Bereich des unteren Sechstels der vorliegenden Trennstufen zuzuführen, wobei hierbei natürlich auch eine direkte Zufuhr in den Sumpf mit umfasst ist.

Alternativ ist es aber auch bevorzugt, in Schritt (c) den genannten Strom aus Schritt (b) enthaltend Ameisensäure und ein tertiäres Amin (I) zum Sumpfverdampfer der Destillationsvorrichtung zuzuführen.

Die Destillationsvorrichtung wird bei einer Sumpftemperatur von 100 bis 300°C und einem Druck von 30 bis 3000 hPa abs betrieben. Bevorzugt wird die Destillationsvorrichtung bei einer Sumpftemperatur von ≥ 120°C, besonders bevorzugt von ≥ 140°C sowie bevorzugt von ≤ 220°C und besonders bevorzugt von ≤ 200°C betrieben. Der Druck beträgt bevorzugt ≥ 30 hPa abs, besonders bevorzugt ≥ 60 hPa abs, sowie bevorzugt ≤ 1500 hPa abs und besonders bevorzugt ≤ 500 hPa abs.

Je nach Zusammensetzung und Ursprung des Ameisensäure und ein tertiäres Amin (I) enthaltenden Zulaufs auf die Destillationsvorrichtung kann Ameisensäure als Kopf- und/oder Seitenprodukt aus der Destillationsvorrichtung erhalten werden. Enthält der Zulauf leichter als Ameisensäure siedende Bestandteile, ist es gegebenenfalls vorteilhaft, diese als Kopfprodukt und die Ameisensäure im Seitenabzug destillativ abzutrennen. Bei im Zulauf möglicherweise gelösten Gasen (wie beispielsweise Kohlenmonoxid oder Kohlendioxid) ist es in der Regel aber auch möglich, die Ameisensäure zusammen mit diesen als Kopfprodukt abzutrennen. Enthält der Zulauf höher als Ameisensäure siedende Bestandteile, wird Ameisensäure bevorzugt als Kopfprodukt, jedoch gegebenenfalls anstelle dessen oder zusätzlich in Form eines zweiten Stroms im Seitenabzug destillativ abgetrennt. Die höher als Ameisensäure siedenden Bestandteile werden in diesem Fall dann bevorzugt über einen zusätzlichen Seitenstrom abgezogen. Der Seitenstrom mit Nebenkomponenten kann ggf. zur Abtrennung der Nebenkomponenten in Schritt (b) zurückgeführt werden.

Auf diese Weise kann Ameisensäure mit einem Gehalt von bis zu 100 Gew.-% gewonnen werden. Im Allgemeinen sind Ameisensäuregehalte von 75 bis 99,995 Gew.-% problemlos erreichbar. Bei dem auf 100 Gew.-% fehlenden Restgehalt handelt es sich hauptsächlich um Wasser, wobei gemäß der in die Destillationsvorrichtung neben Ameisensäure und dem tertiären Amin (I) eingebrachten Stoffen natürlich auch andere Komponenten, wie beispielsweise Lösungsmittel oder auch mögliche Zersetzungsprodukte denkbar sind. So kann Wasser beispielsweise bereits im Zulauf der Destillationsvorrichtung enthalten sein aber gegebenenfalls auch erst bei der thermischen Trennung in geringen Mengen durch Zersetzung von Ameisensäure selbst entstehen.

Bei der Gewinnung von konzentrierter Ameisensäure mit einem Gehalt von 95 bis 100 Gew.-% als Kopf- oder Seitenprodukt wird Wasser mit einem Teil der abgespaltenen Ameisensäure in einem Seitenstrom ausgetragen. Der Ameisensäuregehalt dieses Seitenstroms liegt typischerweise bei 75 bis 95 Gew.-%. Die wässrige Ameisensäure aus dem Seitenstrom kann ggf. zur Abtrennung des Wassers in Schritt (b) zurückgeführt werden.

Es ist aber auch möglich, das Wasser und die abgespaltenen Ameisensäure in einem gemeinsamen Kopf- oder Seitenstrom auszutragen. Der Ameisensäuregehalt des so gewonnen Produkts liegt dann in der Regel bei 85 bis 95 Gew.-%.

Um insbesondere die Bildung organischer Abbauprodukte des tertiären Amins (I) die durch Oxidation entstehen weitestgehend zu unterdrücken ist es vor allem beim Betrieb der Destillationsvorrichtung bei Drücken unterhalb von 0,1 MPa abs besonders vorteilhaft, das Eindringen von Sauerstoff durch eine möglichst geringe Anzahl an Anschlüssen, Stutzen und Flanschen, durch eine besondere Sorgfalt bei der Installation, durch Verwendung von besonders dichten Flanschverbindungen (etwa solche mit Kammprofildichtungen oder Schweißlippendichtungen) oder durch stickstoffabgedeckte Flanschverbindungen zu vermeiden oder zumindest ausgesprochen niedrig zu halten. Eine geeignete Flanschverbindung ist beispielsweise in DE 10 2009 046 310 A1 offenbart.

Die durch das erfindungsgemäße Verfahren erhältliche Ameisensäure besitzt eine niedrige Farbzahl sowie eine hohe Farbzahlstabilität. Im Allgemeinen kann problemlos eine Farbzahl von ≤ 20 APHA, sowie insbesondere sogar von ≤ 10 APHA und gegebenenfalls sogar von ≤ 5 APHA erreicht werden. Selbst bei einer mehrwöchigen Lagerung bleibt die Farbzahl nahezu konstant beziehungsweise nimmt nur unwesentlich zu.

Aufgrund der erfindungsgemäßen Abtrennung der organischen Abbauprodukte des tertiären Amins (I) in Schritt (b) kann ohne weiteren Aufwand eine besonders reine Ameisensäure gewonnen werden, in der die genannten Abbauprodukte im Allgemeinen bei ≤ 70 Gew.-ppm, bevorzugt bei ≤ 30 Gew.-ppm und ganz besonders bevorzugt bei ≤ 20 Gew.-ppm liegen.

Auch der Gehalt an sogenannten Nebenkomponenten ist außerordentlich niedrig und liegt in der Regel bei ≤ 100 Gew.-ppm, bevorzugt bei ≤ 50 Gew.-ppm und ganz besonders bevorzugt bei ≤ 25 Gew.-ppm.

Je nachdem mag es auch von Vorteil sein, in Schritt (c) mehrere Destillationsvorrichtungen einzusetzen, insbesondere wenn neben der freien Ameisensäure und des Amin (I)-haltigen Sumpfprodukts noch weitere Fraktionen, beispielsweise enthaltend Begleitstoffe, Reaktionsnebenprodukte, Verunreinigungen und/oder Ameisensäurefraktionen unterschiedlicher Reinheiten und Konzentrationen, gewonnen werden sollen.

Die Destillationsvorrichtung zur Abtrennung der Ameisensäure kann natürlich auch als thermisch gekoppelte Destillationskolonnen oder als Trennwandkolonne ausgestaltet sein.

In einer bevorzugten Variante des erfindungsgemäßen Verfahrens (i) setzt man in Schritt (a) eine Ameisensäurequelle ein, welche Methylformiat enthält und aus welcher ein flüssiger Strom enthaltend Ameisensäure, tertiäres Amin (I), Wasser und Methanol durch Hydrolyse von Methylformiat erhalten wird, und (ii) trennt in Schritt (b) aus dem aus Schritt (a) erhaltenen Strom einen weiteren Strom enthaltend das aus der Spaltung von Methylformiat entstandene Methanol ab. Das abgetrennte Methanol kann dann beispielsweise erneut in der Synthese von Methylformiat eingesetzt werden. Da Methanol einen deutlich niedrigeren Siedepunkt als Wasser aufweist und somit auch leichter aus einem entsprechenden Gemisch enthaltend Methanol, Wasser, Ameisensäure und tertiären Amin (I) destillativ abtrennbar ist, ist es bei dieser Variante vorteilhaft, Methanol gleich als separaten Strom aus dem aus Schritt (a) erhaltenen Strom abzutrennen.

Wird bei der im vorangehenden Absatz genannten Variante Methanol abgetrennt, ist es besonders vorteilhaft, (i) in Schritt (b) aus dem aus Schritt (a) erhaltenen Strom auch gleich einen weiteren Strom enthaltend nicht umgesetztes Methylformiat abzutrennen und (ii) das abgetrennte Methylformiat zu Schritt (a) rückzuführen. Dadurch kann die Ausbeute an Ameisensäure bezogen auf das eingesetzte Methylformiat deutlich gesteigert werden. Da Methylformiat einen deutlich niedrigeren Siedepunkt als Methanol aufweist und somit noch leichter aus einem entsprechenden Gemisch enthaltend Methylformiat, Methanol, Wasser, Ameisensäure und tertiären Amin (I) destillativ abtrennbar ist, ist es bei dieser Variante vorteilhaft, Methylformiat und Methanol gleich als separate Ströme aus dem aus Schritt (a) erhaltenen Strom abzutrennen. Dies kann beispielsweise in zwei separaten Destillationsvorrichtungen erfolgen, bei denen in der ersten Kolonne Methylformiat und in der zweiten Methanol abgetrennt wird. Es ist aber beispielsweise auch möglich beide Komponenten in einer einzigen Destillationsvorrichtung in separaten Strömen abzutrennen. Methylformiat kann dabei beispielsweise als Kopfprodukt und Methanol als Seitenstromprodukt gewonnen werden.

Die Hydrolyse von Methylformiat in Schritt (a) erfolgt üblicherweise in einem Temperaturbereich von 80 bis 150°C und einem Druckbereich von 0,4 bis 25 MPa abs. Als Vorrichtung zur Durchführung der Hydrolyse in Schritt (a) können prinzipiell alle Vorrichtungen eingesetzt werden, in denen eine exotherme Umsetzung von Fluidströmen möglich ist. Als Beispiele seien etwa Rührkessel, Rohrreaktoren oder Rohrbündelreaktoren, jeweils ohne Einbauten oder mit Einbauten (wie beispielsweise Schüttungen, Füllkörper, Lochbleche und dergleichen) genannt. Die Hydrolyse erfolgt bevorzugt adiabat oder unter Wärmeabfuhr.

In einer anderen bevorzugten Variante des erfindungsgemäßen Verfahrens (i) setzt man in Schritt (a) in Gegenwart von Methanol eine Ameisensäurequelle ein, welche Kohlendioxid, Wasserstoff und einen homogenen Katalysator enthält und aus welcher ein flüssiger Strom enthaltend Ameisensäure, tertiäres Amin (I), Wasser und Methanol durch homogenkatalysierte Hydrierung von Kohlendioxid erhalten wird und (ii) trennt in Schritt (b) aus dem aus Schritt (a) erhaltenen Strom einen weiteren Strom enthaltend Methanol ab und führt das abgetrennte Methanol zu Schritt (a) zurück. Methanol sowie Wasser dienen bei dieser Variante in erster Linie als polare Lösungsmittel.

Die spezifischen Schritte und Verfahrensmerkmale zur homogen-katalysierten Hydrierung von Kohlendioxid zu Ameisensäure in Gegenwart von Wasser und Methanol sind in PCT/EP 2011/060012 (WO 2012/000799) beschrieben.

Als Homogenkatalysator wird dabei bevorzugt eine metallorganische Komplexverbindung enthaltend ein Element aus der 8., 9. oder 10. Gruppe des Periodensystems eingesetzt. Die Komplexverbindung enthält weiterhin bevorzugt mindestens eine Phosphingruppe mit mindestens einem unverzweigten oder verzweigten, acyclischen oder cyclischen aliphatischen Rest mit 1 bis 12 Kohlenstoffatomen, wobei einzelne Kohlenstoffatome auch durch >P- substituiert sein können. Die Hydrierung erfolgt bevorzugt bei 20 bis 200°C sowie bei 0,2 bis 30 MPa abs. Der Austrag aus der Hydrierstufe (a) ist bevorzugt zweiphasig. Die Oberphase enthält tertiäres Amin (I) und Homogenkatalysator, die Unterphase Ameisensäure, tertiäres Amin (I), Wasser, Methanol sowie ebenfalls Homogenkatalysator. Beide Phasen werden getrennt und die tertiäres Amin (I) und Homogenkatalysator enthaltende Oberphase zur Hydrierstufe (a) rückgeführt. Die Unterphase, enthaltend Ameisensäure, tertiäres Amin (I), Wasser, Methanol sowie Homogenkatalysator, wird bevorzugt mit tertiärem Amin (I) extrahiert, um den Großteil des darin vorhandenen Homogenkatalysators zu extrahieren und zusammen mit dem tertiären Amin (I) ebenfalls zur Hydrierstufe (a) rückzuführen. Der verbleibende Rest der Unterphase, welcher Ameisensäure, tertiäres Amin (I), Wasser und Methanol enthält, wird dann Schritt (b) zugeführt, um dann, wie oben beschrieben, Methanol sowie erfindungsgemäß Wasser und organische Abbauprodukte des tertiären Amins (I) abzutrennen.

Auch hinsichtlich der weiteren Aufarbeitung sei ergänzend auf die in PCT/EP 2011/060012 genannten, spezifischen Schritte und Verfahrensmerkmale verwiesen.

In einer bevorzugten Form des erfindungsgemäßen Verfahrens wählt man das in Schritt (a) einzusetzende tertiäre Amin (I) und die Trennrate in der in Schritt (c) genannten Destillationsvorrichtung so, dass sich im Sumpfaustrag

der in Schritt (c) genannten Destillationsvorrichtung zwei flüssige Phasen bilden,

(d) trennt den Sumpfaustrag aus der in Schritt (c) genannten Destillationsvorrichtung in zwei flüssige Phasen, wobei die obere Flüssigphase ein Molverhältnis von Ameisensäure zu tertiärem Amin (I) von 0 bis 0,5 und die untere Flüssigphase ein Molverhältnis von Ameisensäure zu tertiärem Amin (I) von 0,5 bis 4 aufweist;

(e) führt die obere Flüssighase der Phasentrennung aus Schritt (d) zu Schritt (a) zurück; und

(f) führt die untere Flüssigphase der Phasentrennung aus Schritt (d) zu Schritt (b) und/oder (c) zurück.

Die Bildung zweier flüssiger Phasen wird hauptsächlich von den chemischen und physikalischen Eigenschaften der beiden Phasen bestimmt. Diese wiederum können eben durch die Wahl des einzusetzenden tertiären Amins (I), durch die Trennrate in der Destillationsvorrichtung, aber auch durch die Gegenwart eventueller Zusatzkomponenten wie beispielsweise von Lösungsmitteln und deren Konzentrationen beeinflusst werden.

Unter Trennrate ist dabei der Quotient

$$\frac{m_{Ameisensäure}(Zufuhrstrom\ zu\ Schritt\ (c))\,[g/h]\ -\ m_{Ameisensäure}(Sumpfaustrag)\,[g/h]}{m_{Ameisensäure}(Zufuhrstrom\ zu\ Schritt\ (c))\,[g/h]}\cdot 100\,\%$$

zu verstehen, wobei "$m_{Ameisensäure}$(Zufuhrstrom zu Schritt (c))" die pro Zeiteinheit der Destillationsvorrichtung zugeführte Menge an Ameisensäure und "$m_{Ameisensäu\text{-}re}$(Sumpfaustrag)" die pro Zeiteinheit über den Sumpfaustrag abgeführte Menge an Ameisensäure entspricht. Im Allgemeinen wählt man bei dieser bevorzugten Form des erfindungsgemäßen Verfahrens eine Trennrate von ≥ 10%, bevorzugt ≥ 25% und besonders bevorzugt ≥ 40%, sowie im Allgemeinen von ≤ 99,9%, bevorzugt ≤ 99,5% und besonders bevorzugt ≤ 99,0%. Die Trennrate kann beispielsweise einfach durch die Temperatur- und Druckbedingungen in der Destillationsvorrichtung sowie durch die Verweilzeit in der Destillationsvorrichtung beeinflusst werden. Sie kann durch einfache Versuche, gegebenenfalls auch beim Betrieb des erfindungsgemäßen Verfahrens ermittelt werden.

Die Eignung eines tertiären Amins (I) oder eines gegebenenfalls zusätzlich gewünschten Lösungsmittels kann beispielsweise in einfachen Versuchen ermittelt werden, bei dem man unter den beabsichtigten Bedingungen die Phasigkeit ermittelt.

Die Phasentrennung kann beispielsweise in einem separaten Phasenabscheider erfolgen, der der Destillationsvorrichtung nachgeschaltet ist. Es ist aber auch möglich, den Phasenabscheider im Sumpfbereich der Destillationsvorrichtung, im Bereich des Sumpfverdampfers oder auch im Bereich des Sumpfverdampferumlaufs zu integrieren. Hierbei ist beispielsweise auch der Einsatz eines Zentrifugalabscheiders möglich beziehungsweise gegebenenfalls sogar vorteilhaft.

Da die Bildung zweier flüssiger Phasen neben den chemischen und physikalischen Eigenschaften der beiden Phasen zudem auch von der Temperatur beeinflusst wird, wobei in der Regel die Mischbarkeit mit der Temperatur steigt, mag es zur Verbesserung der Phasentrennung gegebenenfalls vorteilhaft sein, diese bei einer tieferen Temperatur als die zuvor gewählte Sumpftemperatur zu betreiben. Dazu wird der Sumpfaustrag üblicherweise in einem zwischengeschalteten Wärmetauscher auf eine Temperatur im Bereich von 30 bis 180°C abgekühlt. Bevorzugt erfolgt die Phasentrennung bei einer Temperatur von ≥ 50°C beziehungsweise bei einer Temperatur von ≤ 160°C und besonders bevorzugt bei einer Temperatur von ≤ 130°C.

Die obere Flüssigphase in Schritt (d) weist ein Molverhältnis von Ameisensäure zu tertiärem Amin (I) im allgemeinen von 0 bis 0,5, bevorzugt von ≥ 0,005 und besonders bevorzugt von ≥ 0,015, sowie von bevorzugt ≤ 0,25 und besonders bevorzugt ≤ 0,125 auf. Die untere Flüssigphase in Schritt (d) weist ein Molverhältnis von Ameisensäure zu tertiärem Amin (I) im allgemeinen von 0,5 bis 4, bevorzugt von ≥ 0,75 und besonders bevorzugt von ≥ 1, sowie von bevorzugt ≤ 3,5 und besonders bevorzugt ≤ 3 auf. Je nach Wahl des Amins kann es jedoch selbstverständlich auch möglich sein, dass die Ameisensäure enthaltende Phase die obere und die Aminphase mit einem molaren Ameisensäure-Aminverhältnis von 0 bis 0,5 die untere Phase bildet. Wichtig ist lediglich, dass es eine Phasentrennung gibt, wobei eine Phase ein Molverhältnis von Ameisensäure zu tertiärem Amin im allgemeinen von 0 bis 0,5 und eine zweite Phase ein Molverhältnis von Ameisensäure zu tertiärem Amin im allgemeinen von 0,5 bis 4 aufweist. Bevorzugt ist die obere Phase jene mit einem Molverhältnis von Ameisensäure zu tertiärem Amin im allgemeinen von 0 bis 0,5 und die untere Phase jene mit einem Molverhältnis von Ameisensäure zu tertiärem Amin im allgemeinen von 0,5 zu 4.

Des Weiteren ist es beim erfindungsgemäßen Verfahren vorteilhaft, die Trennrate in der in Schritt (c) genannten Destillationsvorrichtung so zu wählen, dass das Molverhältnis von Ameisensäure zu tertiärem Amin (I) im Sumpfaustrag 0,1 bis 2,0 beträgt. Unter Sumpfaustrag ist die Gesamtheit der flüssigen Sumpfkondensate zu verstehen, welche die Destillationsvorrichtung verlassen und gemäß Schritt (d) in zwei Flüssigphasen getrennt werden. Dabei ist es unerheblich,

ob die Sumpfkondensate beispielsweise direkt aus dem Sumpf der Destillationsvorrichtung selbst, dem Sumpf des Sumpfverdampfers, oder etwa aus beiden stammen. Bevorzugt wählt man die Trennrate in der in Schritt (c) genannten Destillationsvorrichtung so, dass das Molverhältnis von Ameisensäure zu tertiärem Amin (I) im Sumpfaustrag bevorzugt $\leq$ 1,5 beträgt.

Durch die bevorzugte Rückführung der oberen Flüssigphase der Phasentrennung aus Schritt (d) zu Schritt (a) gemäß Schritt (e) kann das in der oberen Flüssigphase enthaltene tertiäre Amin (I) durch das Zusammenbringen mit der Ameisensäurequelle zur weiteren Erzeugung eines Ameisensäure und tertiäres Amin (I) enthaltenden Stroms eingesetzt werden. Im Allgemeinen führt man 10 bis 100%, bevorzugt 50 bis 100%, besonders bevorzugt 80 bis 100%, ganz besonders bevorzugt 90 bis 100% und insbesondere 95 bis 100% der oberen Flüssigphase zu Schritt (a) zurück.

In der Rückführung der oberen Flüssigphase können selbstverständlich auch weitere Verfahrensschritte integriert sein. Als nicht limitierendes Beispiel sei etwa genannt eine Reinigung der rückzuführenden oberen Flüssigphase beziehungsweise des darin enthaltenen tertiären Amins (I) von unerwünschten Begleitstoffen, Reaktionsnebenprodukten oder Verunreinigungen. Auch hinsichtlich der Art der zwischengeschalteten Verfahrensschritte sind grundsätzlich keine Grenzen gesetzt. Es ist auch möglich, gezielt einen Teil der oberen Flüssigphase als sogenannten "Purge-Strom" zu entfernen. Fehlende beziehungsweise verloren gegangene Mengen an tertiärem Amin (I) können natürlich durch neu zugeführtes tertiäres Amin (I) wieder ergänzt werden, wobei dieses beispielsweise über den Rückführstrom oder auch direkt zu Schritt (a) zugeführt werden kann.

Durch die bevorzugte Rückführung der unteren Flüssigphase der Phasentrennung aus Schritt (d) zu Schritt (b) und/oder (c) gemäß Schritt (f) kann die in der unteren Flüssigphase enthaltene Ameisensäure ebenfalls zur Gewinnung von Ameisensäure durch destillative Abtrennung genutzt werden. Je nach gewünschter Ausführungsform kann die untere Flüssigphase somit (i) zu Schritt (b), (ii) aufgesplittet zu Schritt (b) und (c) oder (iii) zu Schritt (c) rückgeführt werden. Bevorzugt ist im Allgemeinen aber die Rückführung zu Schritt (c), da dadurch die Belastung der Ameisensäure und tertiären Amin (I) enthaltenden unteren Flüssigphase üblicherweise am geringsten ist und der Stoffstrom in Schritt (b) mengenmäßig nicht angehoben wird, was andernfalls eine entsprechend größere Dimensionierung zur Folge haben würde. Im Allgemeinen führt man 10 bis 100%, bevorzugt 50 bis 100%, besonders bevorzugt 80 bis 100%, ganz besonders bevorzugt 90 bis 100% und insbesondere 95 bis 100% der unteren Flüssigphase zu Schritt (b) und/oder (c) zurück.

Es ist aber auch möglich, neben der genannten Rückführung der unteren Flüssigphase zu Schritt (b) und/oder (c), auch einen weiteren Teil zu Schritt (a) rückzuführen. Dies ist beispielsweise im Falle der Erzeugung der Ameisensäure durch übergangsmetallkatalysierte Hydrierung von Kohlendioxid vorteilhaft, da diese im Regelfall in Gegenwart eines polaren Lösungsmittels erfolgt, welches sich ebenfalls in der unteren Flüssigphase anreichert und somit wieder zu Schritt (a) rückgeführt werden kann.

Auch in der Rückführung der unteren Flüssigphase können selbstverständlich noch weitere Verfahrensschritte integriert sein. Als nicht limitierendes Beispiel sei auch hier genannt eine Reinigung der rückzuführenden unteren Flüssigphase beziehungsweise des darin enthaltenen tertiären Amins (I) und/oder der darin enthaltenen Ameisensäure von unerwünschten Begleitstoffen, Reaktionsnebenprodukten oder weiteren Verunreinigungen. Auch hinsichtlich der Art der zwischengeschalteten Verfahrensschritte sind grundsätzlich keine Grenzen gesetzt. Es ist auch möglich, gezielt einen Teil der unteren Flüssigphase als sogenannten "Purge-Strom" auszuschleusen, um damit beispielsweise unerwünschte Begleitstoffe, Reaktionsnebenprodukte oder weitere Verunreinigungen zu entfernen.

Das beim erfindungsgemäßen Verfahren einzusetzende tertiäre Amin (I) weist die allgemeine Formel (I)

$$NR^1R^2R^3 \qquad (I),$$

auf, in der die Reste $R^1$ bis $R^3$ unabhängig voneinander ausgewählt sind aus der Gruppe $C_5$- bis $C_8$-Alkyl.

Als geeignete Amine sind beispielsweise genannt:

- Tri-n-pentylamin, Tri-(3-methylbutyl)-amin, Tri-n-hexylamin, Tri-n-heptylamin, Tri-n-octylamin, Tri-(2-ethylhexyl)amin.
- Di-n-hexyl-(3-methylbutyl)-amin, Di-n-hexyl-(1-methyl-n-hexyl)-amin.

Beim erfindungsgemäßen Verfahren können natürlich auch Gemische verschiedener tertiärer Amine (I) eingesetzt werden. Bevorzugt weisen dann natürlich alle eingesetzten tertiären Amine (I) bei einem Druck von 1013 hPa abs einen um mindestens 5°C höheren Siedepunkt als Ameisensäure auf.

Bevorzugt trägt mindestens einer der Reste am alpha-Kohlenstoffatom, also an dem direkt am Amin-Stickstoffatom gebundenen Kohlenstoffatom, zwei Wasserstoffatome.

Insbesondere setzt man beim erfindungsgemäßen Verfahren als tertiäres Amin (I) Tri-n-pentyl-amin, Tri-n-hexylamin, Tri-n-heptylamin oder Tri-n-octylamin ein.

Die beim erfindungsgemäßen Verfahren gebildeten, Ameisensäure und tertiäres Amin (I) enthaltenden Ströme

können neben der freien Ameisensäure und des freien tertiären Amins (I) im Gemisch die Ameisensäure und das tertiäre Amin (I) auch in verschiedenen anderen Formen enthalten. Die Art und Menge der einzelnen Formen kann dabei je nach den vorliegenden Bedingungen, wie etwa den relativen Mengenverhältnissen von Ameisensäure zu tertiärem Amin (I), der Gegenwart weiterer Komponenten (zum Beispiel Wasser, Lösungsmittel, Nebenprodukte, Verunreinigungen) und somit letztendlich auch der Konzentration von Ameisensäure und tertiärem Amin (I), der Temperatur sowie des Drucks unterschiedlich sein. So seien beispielsweise folgende denkbare Formen erwähnt:

- Ammoniumformiat (Molverhältnis Ameisensäure zu tertiärem Amin (I) von 1) beziehungsweise Ameisensäure-reiches Addukt mit dem tertiären Amin (I) (Molverhältnis Ameisensäure zu tertiärem Amin (I) von > 1).
- Ionische Flüssigkeit.

Für die Durchführung des erfindungsmäßen Verfahrens ist die Art und Menge der einzelnen Formen unerheblich.

Der Schritt (c) zuzuführende flüssige Strom aus Schritt (b) kann neben Ameisensäure und tertiärem Amin (I) natürlich auch noch weitere Komponenten enthalten, wie beispielsweise sogenannte Nebenkomponenten, aber auch Wasser sowie organische Abbauprodukte des tertiären Amins (I), welche in Schritt (b) nicht oder nicht vollständig abgetrennt wurden. Bevorzugt sollten zu Schritt (c) neben Ameisensäure und tertiärem Amin (I) nur solche Komponenten zugeführt werden, welche sich auch in Schritt (c) problemlos von der Ameisensäure destillativ abtrennen lassen oder zumindest in einem nachgeschalteten Schritt von der erhaltenen Ameisensäure leicht abzutrennen sind, wie beispielsweise durch eine nachfolgende Destillation, Extraktion, Absorption oder Adsorption.

Die Konzentration möglicher weiterer Komponenten neben Ameisensäure und tertiärem Amin (I) in dem zu Schritt (c) zuzuführenden flüssigen Stroms beziehungsweise der in diesem Strom befindliche Gehalt an Ameisensäure und tertiärem Amin (I) ist für die Durchführung des erfindungsgemäßen Verfahrens im Allgemeinen grundsätzlich unerheblich, sofern sich die Ameisensäure von diesen in der gewünschten Reinheit problemlos abtrennen lässt. Allerdings ist es aufgrund der Effizienz des erfindungsgemäßen Verfahrens vorteilhaft, die Ameisensäure und das tertiäre Amin (I) nicht in zu großer Verdünnung zu Schritt (c) zuzuführen, da sich eine Verdünnung natürlich in der Regel auch auf die Größe und Auslegung der Destillationsvorrichtung und deren Energieverbrauch niederschlägt. Im Allgemeinen ist es daher ratsam, einen Strom mit mindestens 10 Gew.-% bis 100 Gew.-%, bevorzugt mindestens 50 Gew.-% und besonders bevorzugt mindestens 80 Gew.-% Gesamtgehalt an Ameisensäure und tertiärem Amin (I) zuzuführen.

Der Schritt (c) zuzuführende flüssige Strom aus Schritt (b) kann optional auch sogenannte Lösungsmittel enthalten.

Falls ein Lösungsmittel eingesetzt werden soll, ist es insbesondere bei der bevorzugten Variante, bei der sich im Sumpfaustrag aus der in Schritt (c) genannten Destillationsvorrichtung zwei flüssige Phasen bilden, vorteilhaft, dass dieses nicht oder nur unwesentlich mit dem tertiären Amin (I), aber gut mit der Ameisensäure-haltigen AminPhase mischbar ist und sich in Schritt (d) somit eher in der unteren Flüssigphase wiederfindet. Hierzu hat sich als Maßgröße ein elektrostatischer Faktor, auch EF abgekürzt, von bevorzugt $\geq 200 \cdot 10^{-30}$ Cm, bezogen auf 25°C, herausgestellt. Der elektrostatische Faktor EF ist definiert als das Produkt der relativen Dielektrizitätskonstante $\varepsilon_r$ und dem Dipolmoment $\mu$ des Lösungsmittels (siehe beispielsweise C. Reichardt, "Solvents and Solvent Effects in Organic Chemistry", 3. Auflage, Wiley-VCH Verlag GmbH & Co KGaA, Weinheim 2003, Kapitel 3.2, Seite 67 unten bis Seite 68 oben). Dieser bevorzugte Wert stellt sicher, dass das optionale Lösungsmittel eine gewisse Mindestpolarität aufweist und in Schritt (d) mit der unteren Flüssigphase mischbar ist.

Der Einsatz von Lösungsmitteln kann, abhängig vom jeweiligen System (zum Beispiel Art des tertiären Amins (I), Konzentrationen, Temperatur, Druck und dergleichen), beispielsweise die Trennung der beiden Flüssigphasen verbessern.

Als Stoffklassen, die als optionales Lösungsmittel besonders geeignet sind, kommen insbesondere Ameisensäureester, Diole sowie deren Ameisensäureester, Polyole sowie deren Ameisensäureester, Sulfone, Sulfoxide, offenkettige oder cyclische Amide sowie Gemische der genannten Stoffklassen in Frage.

Als geeignete Diole und Polyole sind beispielsweise Ethylenglykol (EF = $290{,}3 \cdot 10^{-30}$ Cm), Diethylenglykol (EF = $244{,}0 \cdot 10^{-30}$ Cm), Triethylenglykol, Polyethylenglykol, 1,3-Propandiol (EF = $285{,}6 \cdot 10^{-30}$ Cm), 2-Methyl-1,3-propandiol, 1,4-Butandiol (EF = $262{,}7 \cdot 10^{-30}$ Cm), Dipropylenglykol, 1,5-Pentandiol (EF = $212{,}5 \cdot 10^{-30}$ Cm), 1,6-Hexandiol und Glycerin genannt. Diole und Polyole können aufgrund ihrer OH-Gruppen in Gegenwart von Ameisensäure verestert werden. Dies geschieht beim erfindungsgemäßen Verfahren vor allem in Schritt (c) bei der thermischen Trennung des Ameisensäure und tertiäres Amin (I) enthaltenden Stroms in der genannten Destillationsvorrichtung. Da die entstehenden Ameisensäureester ein sehr ähnliches Phasenverhalten zeigen, sind diese als Lösungsmittel im Allgemeinen ebenso gut geeignet. Auch das bei der Veresterung entstehende Wasser ist bei der thermischen Trennung unschädlich. Eine Aufpegelung des Wassers im kontinuierlichen Betrieb des erfindungsgemäßen Verfahrens erfolgt nicht, da Wasser in diesen geringen Mengen in der Destillationsvorrichtung über einen Seitenabzug abgetrennt werden kann.

Als geeignete Sulfoxide sind beispielsweise Dialkylsulfoxide, vorzugsweise $C_1$- bis $C_6$-Dialkylsulfoxide, insbe-

sondere Dimethylsulfoxid (EF = 627,1 · $10^{-30}$ Cm) genannt.

Als geeignete offenkettige oder cyclische Amide sind beispielsweise Formamid (EF = 1243,2 · $10^{-30}$ Cm), N-Methylformamid (EF = 2352,9 · $10^{-30}$ Cm), N,N-Dimethylformamid (EF = 396,5 · $10^{-30}$ Cm), N-Methylpyrrolidon (EF = 437,9 · $10^{-30}$ Cm), Acetamid und N-Methylcaprolactam genannt.

Je nachdem kann es aber auch vorteilhaft sein, gerade ein eher unpolares Lösungsmittel mit < 200 x $10^{-30}$ Cm, bezogen auf 25°C, einzusetzen. Unpolare Lösungsmittel können gegebenenfalls die Konzentration an Ameisensäure in der oberen Flüssigphase verringern.

Bevorzugt wird das erfindungsgemäße Verfahren jedoch ohne Zusatz eines Lösungsmittels durchgeführt.

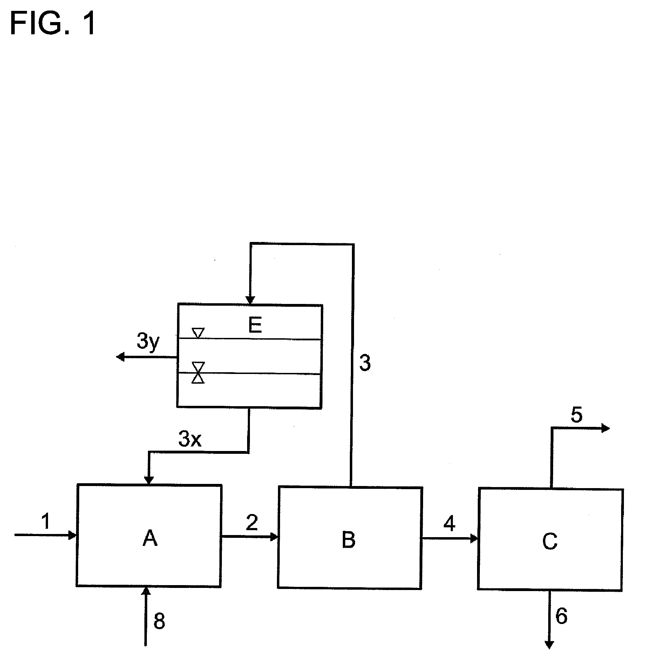

Fig. 1 zeigt ein vereinfachtes Blockdiagramm einer allgemeinen Ausführungsform des erfindungsgemäßen Verfahrens. Darin haben die einzelnen Buchstaben folgende Bedeutung:

A = Vorrichtung zur Erzeugung eines Ameisensäure, tertiäres Amin (I) und Wasser enthaltenden Stroms
B = Vorrichtung zur Abtrennung von Wasser, organischen Abbauprodukten des tertiären Amins (I) und gegebenenfalls sogenannten Nebenprodukten
C = Destillationsvorrichtung
E = Phasentrenngefäß

Über Strom (1) werden Wasser und eine Ameisensäurequelle und über Strom (8) tertiäres Amin (I) der Vorrichtung A zur Erzeugung eines Ameisensäure, tertiäres Amin (I) und Wasser enthaltenden Stroms zugeführt. Der Ameisensäure, tertiäres Amin (I) und Wasser enthaltende Strom (2) wird aus Vorrichtung A abgezogen und zur Abtrennung von Wasser und organischen Abbauprodukten des tertiären Amins (I) der Vorrichtung B zugeführt. Bei dieser kann es sich beispielsweise um eine Destillationsvorrichtung handeln. Abgetrenntes Wasser und organische Abbauprodukte des tertiären Amins (I) werden über Strom (3) entnommen und dem Phasentrenngefäß E zugeführt. Darin bilden sich zwei Flüssigphasen aus. Die untere, Wasser enthaltende Flüssigphase wird als Strom (3x) zur Vorrichtung A rückgeführt. Die obere, an organischen Abbauprodukten des tertiären Amins (I) angereicherte Flüssigphase wird als Strom (3y) entnommen und aus dem Verfahren ausgeschleust. Über Strom (4) wird der an Ameisensäure und tertiärem Amin (I) aufkonzentrierte Strom der Destillationsvorrichtung C zugeführt. Darin erfolgt die destillative Abtrennung von Ameisensäure als Strom (5). Der Sumpf der Destillationsvorrichtung C wird als Strom (6) entnommen.

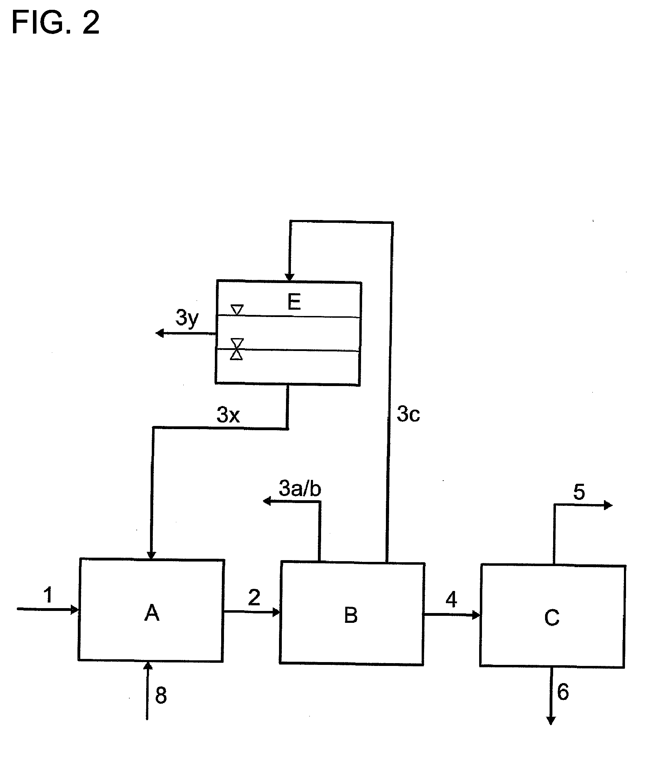

Fig. 2 zeigt ein vereinfachtes Blockdiagramm einer abgewandelten Ausführungsform, bei der in Schritt (b) des erfindungsgemäßen Verfahrens neben Wasser und organischen Abbauprodukten des tertiären Amins (I) über Strom (3a/b) noch weitere, sogenannte Nebenprodukte abgetrennt werden. Die Vorrichtungen A, B, C und E haben dabei die bei Fig. 1 genannten Bedeutungen. Bei Vorrichtung B kann es sich beispielsweise um zwei hintereinandergeschaltete Destillationsvorrichtungen handeln. Ebenso ist aber auch denkbar, Vorrichtung B als eine einzige Destillationsvorrichtung auszugestalten, in der beispielsweise der Strom (3a/b) als Kopfstrom und der Strom (3c) als Seitenstrom entnommen werden.

In einer von Fig. 2 geringfügig abweichenden Variante werden anstelle eines gemeinsamen Stroms (3a/b) in Vorrichtung B zwei getrennte Ströme als Strom (3a) und (3b) entnommen.

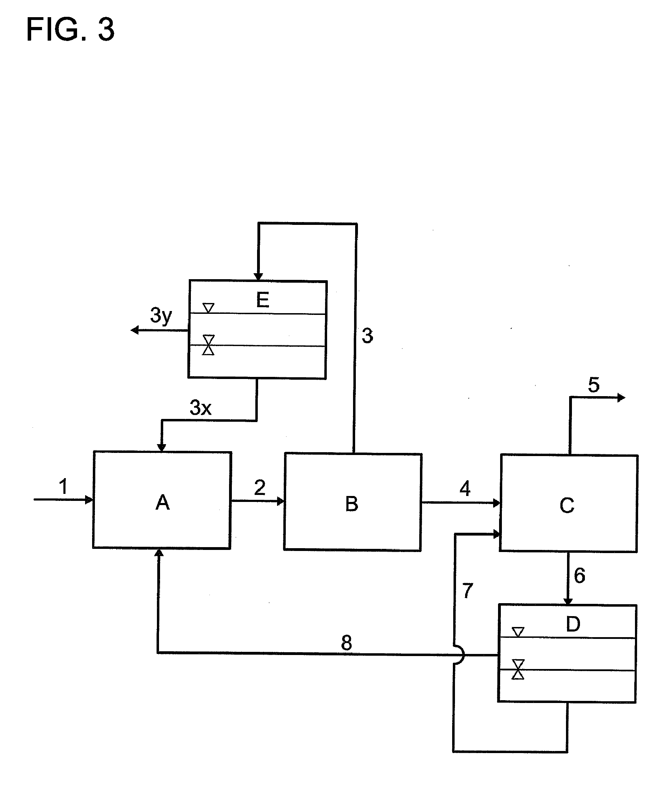

In Fig. 3 ist das vereinfachte Blockdiagramm einer bevorzugten Ausführungsform mit separater Rückführung des Sumpfaustrags aus der Destillationsvorrichtung C. Die Vorrichtungen A, B, C und E haben dabei wieder die bei Fig. 1 genannten Bedeutungen.

Weiterhin umfasst Fig. 3 noch folgende Vorrichtungen:

D = Phasentrenngefäß

Gegenüber dem vereinfachten Blockdiagramm von Fig. 1 wird im Falle von Fig. 3 der Sumpf der Destillationsvorrichtung C als Strom (6) dem Phasentrenngefäß D zugeführt und in zwei flüssigen Phasen getrennt. Die obere Phase wird als Strom (8) zur Vorrichtung A rückgeführt. Die untere Phase wird als Strom (7) zur Destillationsvorrichtung C rückgeführt.

In einer alternativen Ausführungsform kann das Phasentrenngefäß D auch in die Destillationsvorrichtung C integriert sein.

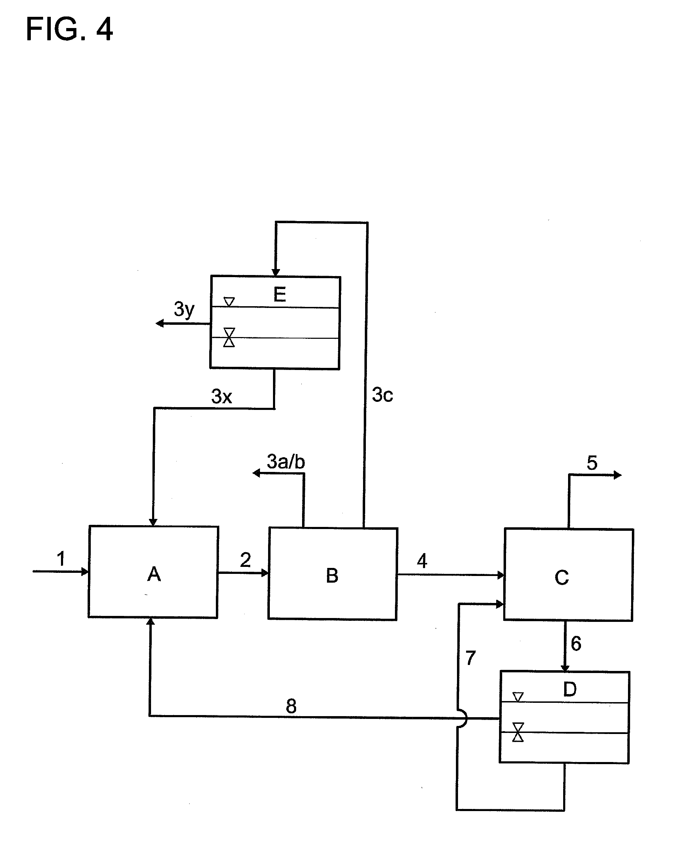

Fig. 4 zeigt ein vereinfachtes Blockdiagramm einer gegenüber Fig. 3 abgewandelten Ausführungsform, bei der in Schritt (b) des erfindungsgemäßen Verfahrens neben Wasser und organischen Abbauprodukten des tertiären Amins (I) über Strom (3a/b) noch weitere, sogenannte Nebenprodukte abgetrennt werden. Hinsichtlich der Abtrennung von Nebenprodukten sei auch auf die Erläuterungen zu Fig. 2 verwiesen.

Im Bereich der Destillationsvorrichtung C und der Phasentrennung D sind verschiedene Ausgestaltungsformen möglich. Sie unterscheiden sich nicht nur darin, ob die Phasentrennung in einem separaten Behälter oder integriert im Sumpf der Destillationskolonne erfolgt, sondern auch im Ort der Zugabe des Ameisensäure und tertiären Amin (I) enthaltenden Stroms zur Destillationsvorrichtung und in der Stromführung zwischen dem Kolonnenbehälter und dem

Sumpfverdampfer sowie der Entnahmestelle des Sumpfaustrags. Die in PCT/EP-Az. 2011/060,770 in Fig. 2 bis 7 dargestellten und im Text beschriebenen Ausführungsformen sind auch im Rahmen des bevorzugten, erfindungsgemäßen Verfahrens anwendbar.

Im Folgenden sind zweibevorzugte Ausführungsformen zu bevorzugten Anwendungsfeldern des erfindungsgemäßen Verfahrens beschrieben.

Gewinnung von Ameisensäure durch Hydrolyse von Methylformiat

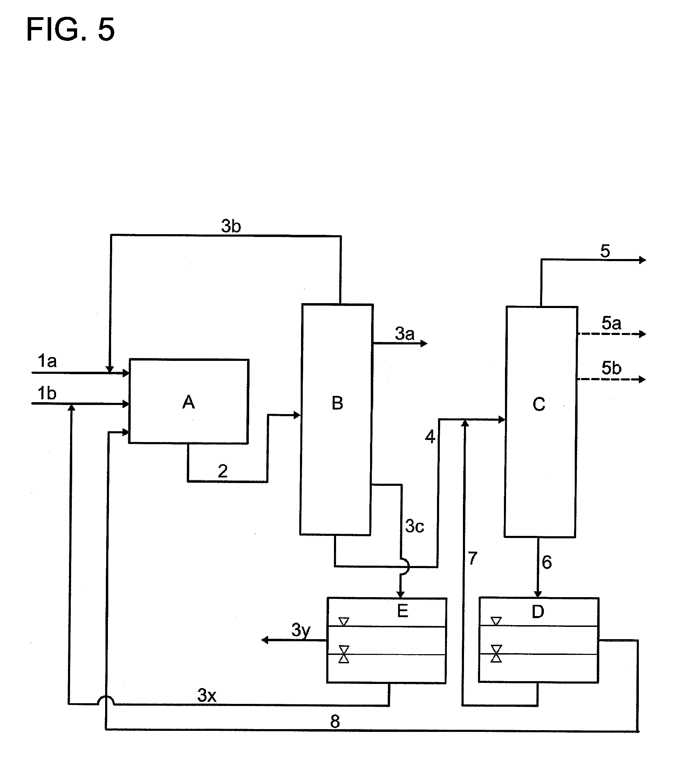

Eine bevorzugte Ausführungsform zur Gewinnung von Ameisensäure durch Hydrolyse von Methylformiat ist in Fig. 5 durch ein vereinfachtes Blockdiagramm wiedergegeben.

Darin haben die einzelnen Buchstaben folgende Bedeutung:

- A = Vorrichtung zur Hydrolyse von Methylformiat und Erzeugung eines Ameisensäure, tertiäres Amin (I) und Wasser enthaltenden Stroms
- B = Destillationsvorrichtung zur Abtrennung von Methylformiat, Methanol, Wasser und organischen Abbauprodukten des tertiären Amins (I)
- C = Destillationsvorrichtung zur Gewinnung von Ameisensäure
- D = Phasentrenngefäß
- E = Phasentrenngefäß

Methylformiat (Ströme (1a) und (3b)), Wasser (Ströme (1b) und (3x)) sowie tertiäres Amin (I) (Strom (8)) werden der Vorrichtung A zugeführt. Durch Hydrolyse von Methylformiat entsteht ein Ameisensäure, tertiäres Amin (I), Methanol, Wasser und Methylformiat enthaltender Strom, der als Strom (2) aus der Vorrichtung A entnommen und der Vorrichtung B zugeführt wird. Der Methylformiat-Umsatz und somit die Zusammensetzung des Stroms (2) hängt in erster Linie von den relativen Zulaufmengen der drei Feedströme Methylformiat, Wasser und tertiäres Amin (I) zur Vorrichtung A, der Art des eingesetzten tertiären Amins (I), der Verweilzeit und der Reaktionstemperatur ab. Die für das jeweilige Reaktionssystem sinnvollen Bedingungen können vom Fachmann beispielsweise durch Vorversuche leicht ermittelt werden. In Strom (2) beträgt das Molverhältnis von Ameisensäure zu tertiärem Amin (I) üblicherweise 0,5 bis 5, bevorzugt 0,5 bis 3, wobei natürlich auch Abweichungen von diesen Bereichen möglich sind.

In der Destillationsvorrichtung B werden von Strom (2) nun nicht umgesetztes Methylformiat (Strom (3b)), bei der Hydrolyse gebildetes Methanol (Strom (3a)) sowie Wasser und organische Abbauprodukte des tertiären Amins (I) (Strom (3c)) abgetrennt. Strom (3b), enthaltend den nicht umgesetzten Einsatzstoffe Methylformiat, wird zur Vorrichtung A rückgeführt. Das über Strom (3a) abgetrennte Methanol kann beispielsweise erneut zur Herstellung von Methylformiat eingesetzt werden. Strom (3c), enthaltend Wasser und organische Abbauprodukte des tertiären Amins (I), wird dem Phasentrenngefäß E zugeführt und in zwei Flüssigphasen getrennt. Die untere Phase enthaltend Wasser wird als Strom (3x) ebenfalls zur Vorrichtung A rückgeführt. Die obere Phase enthaltend organische Abbauprodukte des tertiären Amins (I) wird aus dem Verfahren ausgeschleust. Ameisensäure und tertiäres Amin (I) werden via Strom (4) entnommen. Dieser enthält zudem noch Restmengen an Wasser. Je nach Ausführung des Verfahrens können diese einige Gewichtsprozente oder sogar einige zehn Gewichtsprozente des Stroms (4) ausmachen. Bevorzugt liegt der Wassergehalt in Strom (4) bei $\leq$ 20 Gew.-%, besonders bevorzugt bei $\leq$ 10 Gew.-% und ganz besonders bevorzugt bei $\leq$ 5 Gew.-%. Das Molverhältnis von Ameisensäure zu tertiärem Amin (I) wird durch die Destillationsvorrichtung B nicht oder nur unwesentlich verändert, so dass dieses auch in Strom (4) üblicherweise 0,5 bis 5, bevorzugt 0,5 bis 3, beträgt, wobei natürlich auch Abweichungen von diesen Bereichen möglich sind.

Strom (4) wird der Destillationsvorrichtung C zugeführt. Darin wird die Ameisensäure über Strom (5) als Kopfprodukt, über Strom (5a) als Seitenprodukt und/oder über Strom (5b) als Seitenprodukt destillativ entfernt. Je nach den Rahmenbedingungen, das heißt vor allem der Zusammensetzung des Zufuhrstroms (4) zur Destillationsvorrichtung C sowie der gewünschten Reinheit der Ameisensäure, kann in der vorliegenden Ausführungsform Ameisensäure als Strom (5) über Kopf oder als Strom (5a) als Seitenprodukt erhalten werden. Wasserhaltige Ameisensäure wird dann als Seitenprodukt über Strom (5a) beziehungsweise (5b) entnommen. Im Einzelfall mag es sogar ausreichen, Ameisensäure beziehungsweise wasserhaltige Ameisensäure lediglich über Strom (5) als Kopfprodukt zu entfernen. Je nach konkreter Ausführungsform kann somit der Seitenstrom (5b) oder sogar beide Seitenströme (5a) und (5b) entfallen. Die Destillationsvorrichtung C kann natürlich auch die in Fig. 2 bis 7 der PCT/EP-Az. 2011/060,770 offenbarten Ausgestaltungsformen aufweisen.

Das Sumpfprodukt der Destillationsvorrichtung C wird als Strom (6) dem Phasentrenngefäß D zugeführt. Alternativ kann der Phasenabscheider D auch in die Destillationsvorrichtung C integriert sein. Im Phasentrenngefäß D wird das Sumpfprodukt in zwei Flüssigphasen getrennt. Zwischen der Destillationsvorrichtung C und dem Phasentrenngefäß D kann optional zur Abkühlung des entnommenen Sumpfstroms beispielsweise auch ein Wärmetauscher zwischengeschaltet sein. Eine niedrigere Phasentrenntemperatur führt zwar im Regelfall zu einer etwas besseren Trennung hinsichtlich des Ameisensäuregehalts, verursacht jedoch aufgrund des Einsatzes eines Wärmetauschers zusätzlichen Aufwand und Energieverbrauch. Vor- und Nachteile sind daher jeweils abzuwägen. Die obere Flüssigphase aus dem

Phasentrenngefäß D wird über Strom (8) zur Vorrichtung A rückgeführt. Die untere Flüssigphase wird über Strom (7) zur Destillationsvorrichtung C rückgeführt.

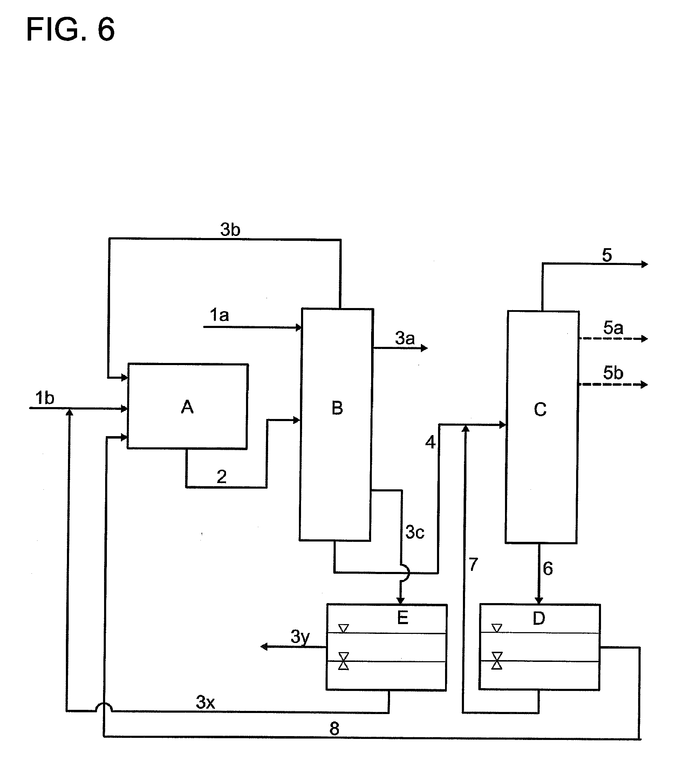

Bei einer anderen, bevorzugten Ausführungsform zur Gewinnung von Ameisensäure durch Hydrolyse von Methylformiat wird gemäß Fig. 6 der Methylformiat-Strom (1a) in die Destillationsvorrichtung B gegeben. Diese Ausführungsform ist im Allgemeinen vorteilhaft, wenn das als Strom (1 a) zur Verfügung stehende Methylformiat noch mit Restmengen an Methanol verunreinigt ist, beispielsweise durch eine vorangestellte Methylformiat-Synthesestufe mit Methanol-Teilumsatz und unvollständiger Aufarbeitung des Methylformiats. Durch die direkte Zufuhr von Strom (1 a) in die Destillationsvorrichtung B kann somit das enthaltene Methanol als Strom (3a) abgetrennt und beispielsweise zur Methylformiat-Synthesestufe rückgeführt werden. Durch diese Variante ist es möglich, in der Methylformiat-Synthesestufe sogar ganz auf eine Methylformiat/Methanol-Trennung zu verzichten und somit eine ganze Destillationskolonne und somit im laufenden Betrieb auch Energie einzusparen.

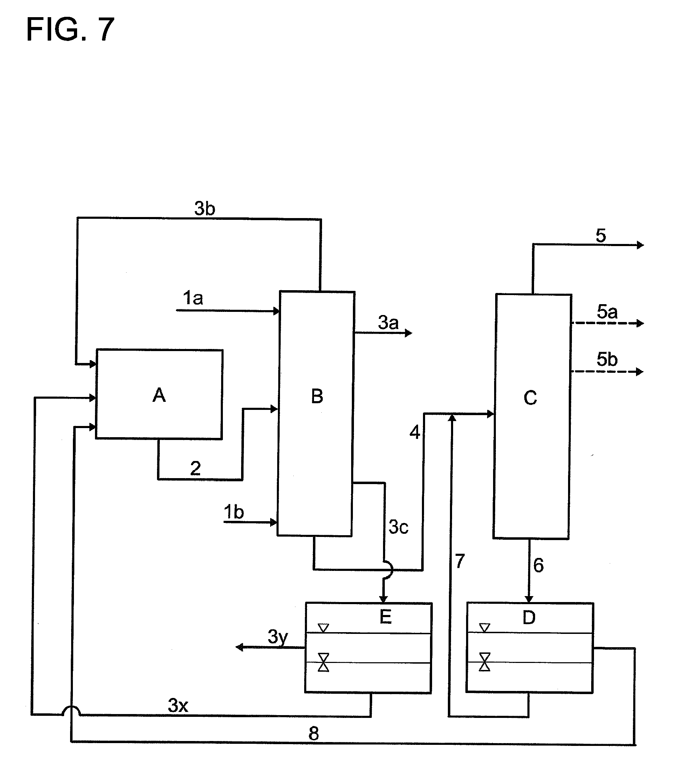

Bei einer weiteren, bevorzugten Ausführungsform zur Gewinnung von Ameisensäure durch Hydrolyse von Methylformiat wird gemäß Fig. 7 sowohl der Methylformiat-Strom (1 a) als auch der Wasser-Strom (1 b) in die Destillationsvorrichtung B gegeben. Hinsichtlich des Wasser-Stroms (1 b) ist diese Ausführungsform im Allgemeinen vorteilhaft, wenn Heißkondensat oder Dampf als Wasserquelle zur Verfügung steht, da hierdurch die darin gespeicherte thermische Energie in der Destillationsvorrichtung B genutzt werden kann.

Der Vollständigkeit halber sei noch erwähnt, dass es in einer weiteren Ausführungsform natürlich auch möglich ist, den Methylformiat-Strom (1a) in die Vorrichtung A zu geben, den Wasser-Strom (1 b) jedoch in die Destillationsvorrichtung B. Dies ist beispielsweise vorteilhaft, wenn Niederdruck-Überschussdampf zur Verfügung steht.

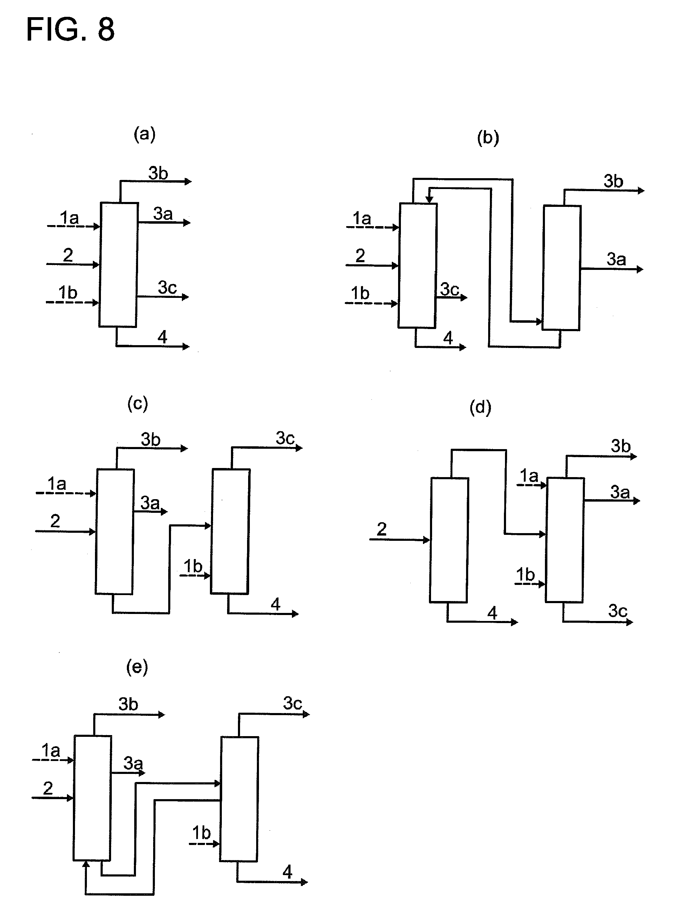

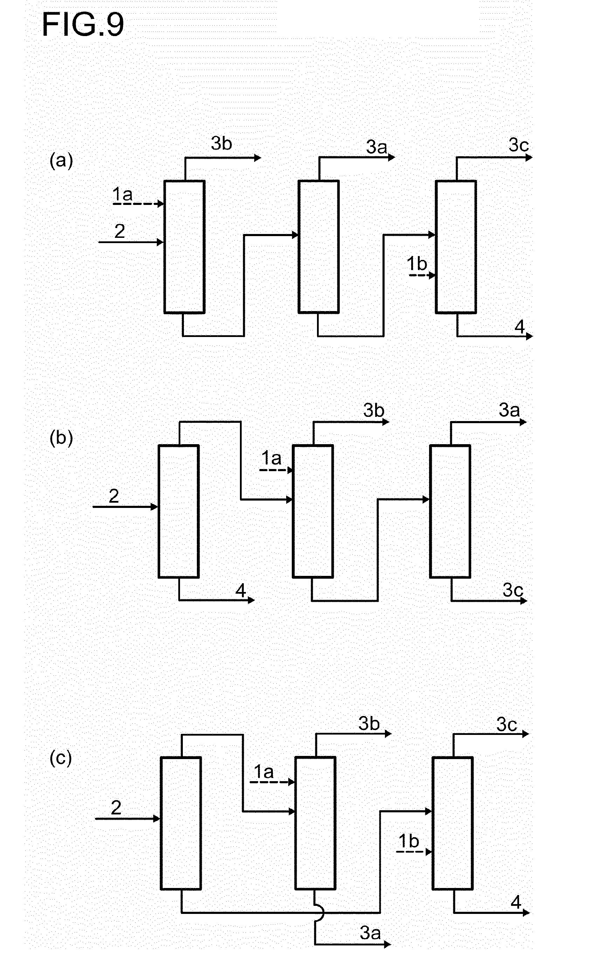

Bei den Varianten der Fig. 5 bis 7 sind hinsichtlich der Ausgestaltungsform der Destillationsvorrichtung B spezifische Varianten mit einer, zwei oder sogar drei Destillationskolonnen möglich. Fig. 8a zeigt eine Ausgestaltungsform mit einer Destillationskolonne. Fig. 8b bis 8e zeigen verschiedene Ausgestaltungsformen mit zwei Destillationskolonnen. Fig. 9a bis 9c zeigen verschiedene Ausgestaltungsformen mit drei Destillationskolonnen. Bevorzugt zur Ausgestaltung der Destillationsvorrichtung B sind die Varianten mit einer oder zwei Destillationskolonnen. Der Vollständigkeit halber sei erwähnt, dass insbesondere bei den Ausgestaltungsformen mit einer oder zwei Destillationskolonnen diese auch als thermisch gekoppelte oder Trennwandkolonne ausgestaltet sein können.

Gewinnung von Ameisensäure durch Hydrierung von Kohlendioxid

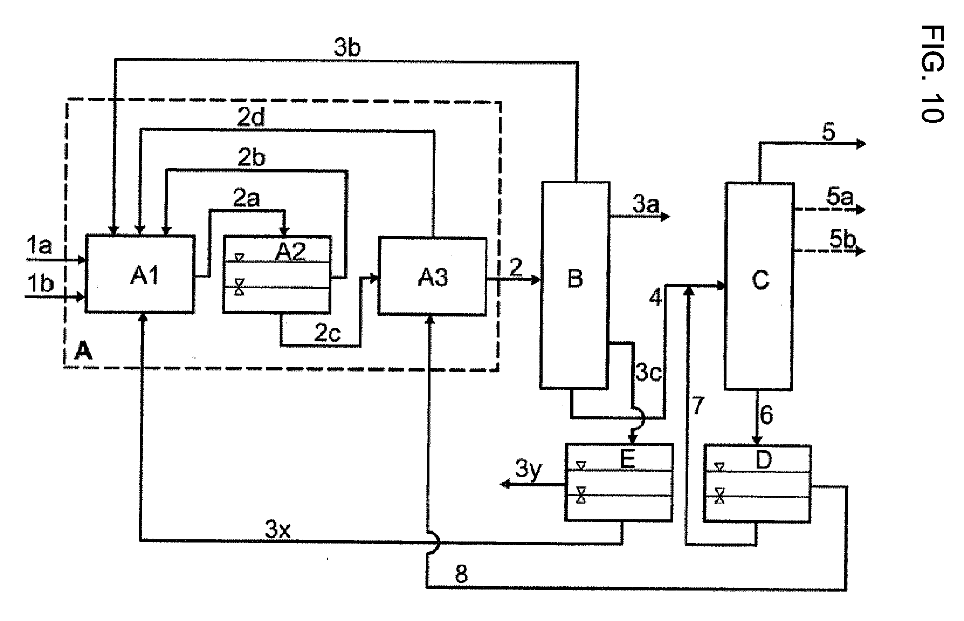

Eine bevorzugte Ausführungsform zur Gewinnung von Ameisensäure durch Hydrierung von Kohlendioxid ist in Fig. 10 durch ein vereinfachtes Blockdiagramm wiedergegeben. Darin haben die einzelnen Buchstaben folgende Bedeutung:

- A = Vorrichtung zur Hydrierung von Kohlendioxid und Erzeugung eines Ameisensäure, tertiäres Amin (I) und Wasser enthaltenden Stroms
- A1 = Hydrierreaktor
- A2 = Phasentrenngefäß
- A3 = Extraktionseinheit
- B = Destillationsvorrichtung zur Abtrennung von Methanol, Wasser und organischen Abbauprodukten des tertiären Amins (I)
- C = Destillationsvorrichtung zur Gewinnung von Ameisensäure
- D = Phasentrenngefäß
- E = Phasentrenngefäß

Kohlendioxid (Strom (1 a)), Wasserstoff (Strom (1 b)) sowie tertiäres Amin (I) (Strom (2d)) werden dem Hydrierreaktor A1 in Vorrichtung A zugeführt. Im Hydrierreaktor A1 erfolgt in Gegenwart eines Homogenkatalysators sowie von Wasser und Methanol als Lösungsmittel die Hydrierung unter Bildung eines Ameisensäure, tertiäres Amin (I), Methanol, Wasser und Homogenkatalysator enthaltenden Stroms (2a). Dieser wird dem Phasentrenngefäß A2 zugeführt, in dem sich zwei flüssige Phasen bilden. Die obere Flüssigphase, enthaltend tertiäres Amin (I) und Homogenkatalysator, wird über Strom (2b) zum Hydrierreaktor A1 rückgeführt. Die untere Flüssigphase, enthaltend Ameisensäure, tertiäres Amin (I), Wasser, Methanol sowie ebenfalls Homogenkatalysator, wird über Strom (2c) zur Extraktionseinheit A3 geleitet. In dieser werden über das als Strom (8) zugeführte tertiäre Amin (I) die Reste des noch vorhandenen Homogenkatalysators weitgehend extrahiert und zusammen mit dem tertiären Amin (I) als Strom (2d) zum Hydrierreaktor A1 rückgeführt. Als Strom (2) wird somit ein Ameisensäure, tertiäres Amin (I) und Wasser enthaltender Strom erhalten und der Destillationsvorrichtung B zugeführt.

In der Destillationsvorrichtung B werden von Strom (2) Methanol (Strom (3b)) sowie Wasser und organische Abbauprodukte des tertiären Amins (I) (Strom (3c)) abgetrennt. Strom (3b), enthaltend Methanol, wird zum Hydrierreaktor A1 in Vorrichtung A rückgeführt. Strom (3c), enthaltend Wasser und organische Abbauprodukte des tertiären Amins (I),

wird dem Phasentrenngefäß E zugeführt und in zwei Flüssigphasen getrennt. Die untere Phase enthaltend Wasser wird als Strom (3x) ebenfalls zum Hydrierreaktor A1 in Vorrichtung A rückgeführt. Die obere Phase enthaltend organische Abbauprodukte des tertiären Amins (I) wird aus dem Verfahren ausgeschleust. Ameisensäure und tertiäres Amin (I) werden via Strom (4) entnommen und zur Destillationsvorrichtung C geleitet. Hinsichtlich der Verfahrensschritte betreffend die Destillationsvorrichtung C und das Phasentrenngefäß D sei auf die obige Beschreibung zur Gewinnung von Ameisensäure durch Hydrolyse von Methylformiat verwiesen.

Das erfindungsgemäße Verfahren ermöglicht die Gewinnung von Ameisensäure in hoher Ausbeute und hoher Konzentration durch thermische Trennung eines Stroms enthaltend Ameisensäure und ein tertiäres Amin.

Durch die erfindungsgemäße Abtrennung von Wasser und organischen Abbauprodukten des tertiären Amins (I) mit nachfolgender Phasentrennung des abgetrennten Stroms in eine Wasser enthaltende Flüssigphase und eine organische Abbauprodukte des tertiären Amins (I) enthaltende Flüssigphase ermöglicht das erfindungsgemäße Verfahren auch über längere Betriebszeiten hinweg einen sehr stabilen Betrieb bei gleichzeitig konstant hoher Reinheit der produzierten Ameisensäure. Die zu gewinnende Ameisensäure besitzt eine niedrige Farbzahl sowie eine hohe Farbzahlstabilität.

Das Verfahren ist einfach, zuverlässig und auch energiegünstig durchführbar, zumal durch die überraschend gefundene Möglichkeit, die organischen Abbauprodukte des tertiären Amins (I) zusammen mit dem Wasser abzutrennen und daraus durch Phasentrennung zu isolieren nur einen sehr geringen zusätzlichen Aufwand in Form der Bereitstellung einer geeigneten Phasentrennvorrichtung erfordert. Durch die geschickte erfindungsgemäße Maßnahme werden im Gegensatz zu herkömmlichen Isolierungen und Ausschleusungen störender Neben- oder Abbauprodukte weder aufwändige zusätzliche Vorrichtungen noch signifikante Mengen an zusätzlicher Energie benötigt.

Das erfindungsgemäße Verfahren ist insbesondere auch besonders vorteilhaft in Verbindung mit der Hydrolyse von Methylformiat als Ameisensäurequelle einsetzbar und besitzt gegenüber den derzeit großtechnisch ausgeübten Betriebsverfahren der Methylformiat-Hydrolyse mit nachgeschalteter Entwässerung mittels eines Extraktionshilfsmittels oder einer Zweidruckdestillation technische und wirtschaftliche Vorteile.

## Beispiele

### Laboranlage 1 (für Vergleichsbeispiel 1)

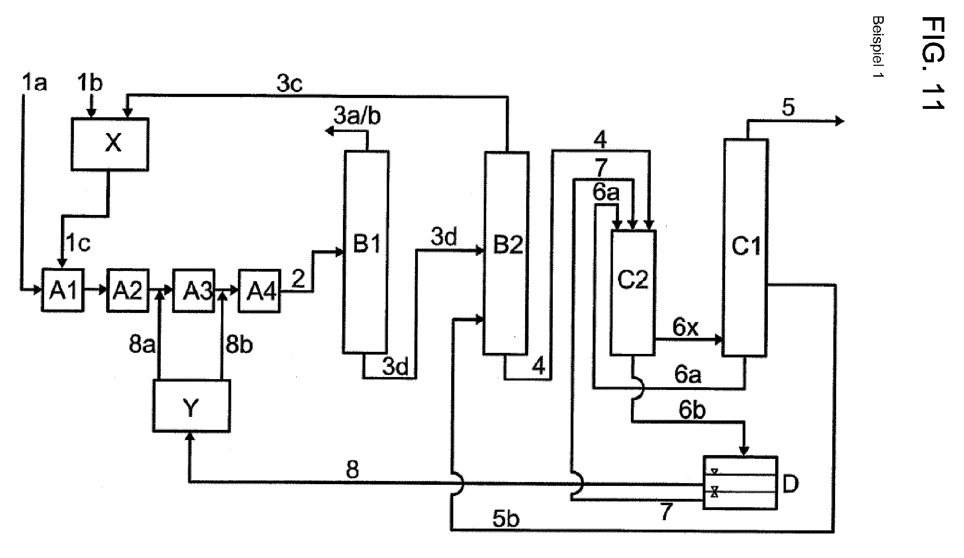

Laboranlage 1 diente zur Untersuchung des kontinuierlichen Verfahrens ohne Anwendung der vorliegenden Erfindung. Das vereinfachte Blockdiagramm von Laboranlage 1 ist in Fig. 11 dargestellt. Darin haben die einzelnen Buchstaben folgende Bedeutung:

| | |
|---|---|
| A1 | = Rührkessel (Volumen 0,3 L, elektrisch beheizt) |
| A2,3,4 | = jeweils Rohrreaktor (Innendurchmesser 80 mm, Länge 1200 mm, gefüllt mit 2 mm-Glaskugeln, elektrisch beheizt) |
| X | = Mischbehälter (Volumen 5 L) |
| Y | = Behälter (Volumen: 5 L) |
| B1 | = Destillationsvorrichtung mit Kolonnenkörper (Innendurchmesser 55 mm, bestückt mit zwei Gewebepackungen mit je 1,3 m Packungshöhe und einer spezifischen Oberfläche von 750 $m^2/m^3$, wobei sich der Zulauf von Strom (2) zwischen den beiden Gewebepackungen befand), ölbeheiztem Fallfilmverdampfer und Kondensator sowie regelbarem Rücklaufteiler am Kolonnenkopf |
| B2 | = Destillationsvorrichtung mit Kolonnenkörper (Innendurchmesser 55 mm, bestückt mit 12 Glockenböden im Abtriebsteil und 10 Glockenböden im Verstärkungsteil, wobei sich der Zulauf von Strom (3d) zwischen beiden Teilen und der Zulauf von Strom (5b) im Abtriebsteil befand), ölbeheiztem Fallfilmverdampfer und Kondensator sowie regelbarem Rücklaufteiler am Kolonnenkopf |
| C1 | = Kolonnenkörper (Innendurchmesser 43 mm, bestückt mit einer Gewebepackung oberhalb des Sumpfes mit einer Packungshöhe von 0,66 m und einer spezifischen Oberfläche von 500 $m^2/m^3$ sowie einer weiteren Gewebepackung mit einer Packungshöhe von 1,82 m und einer spezifischen Oberfläche von 750 $m^2/m^3$, wobei sich der Seitenabzug von Strom (5b) zwischen beiden Gewebepackungen befand) und Kondensator sowie regelbarem Rücklaufteiler am Kolonnenkopf |
| C2 | = Ölbeheizter Fallfilmverdampfer |
| D | = Separates Phasentrenngefäß (Volumen 0,3 L, ölbeheizt) |

Die Apparate und Leitungen bestanden aus einer Nickelbasislegierung mit der Werkstoffnummer 2.4610. Die Erfassung der Massenströme erfolgte mit einem Coriolis-Durchflussmesser. Laboranlage 1 wurde kontinuierlich betrieben.

Bei allen Versuchen in der Laboranlage 1 wurde der Gehalt an Ameisensäure jeweils durch potentiometrische

Titration mit 0,5 N NaOH in Wasser und der Gehalt an Wasser nach Karl Fischer bestimmt. Alle anderen organischen Komponenten wurden jeweils gaschromatographisch bestimmt.

Laboranlage 2 (für erfindungsgemäßes Beispiel 2)

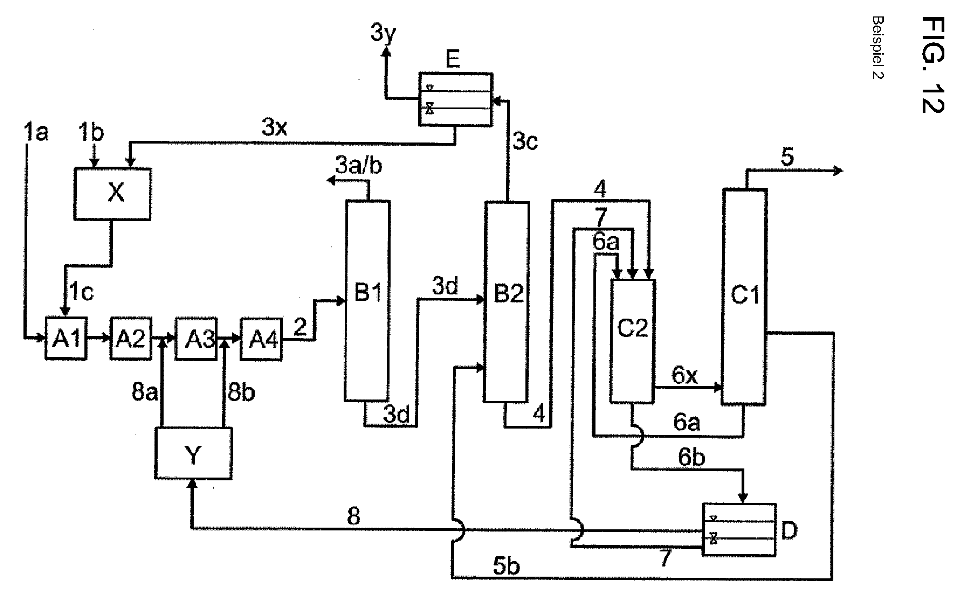

Laboranlage 2 ist die um ein separates Phasentrenngefäß für Strom (3c) erweiterte Laboranlage 1 und diente zur Untersuchung des kontinuierlichen Verfahrens unter Anwendung der vorliegenden Erfindung. Das vereinfachte Blockdiagramm von Laboranlage 2 ist in Fig. 12 dargestellt. Darin hat der ergänzte Buchstabe folgende Bedeutung:

E = Separates Phasentrenngefäß (Volumen 25 mL, ölbeheizt)

Im Übrigen wird auf die Beschreibung der Laboranlage 1 verwiesen.

Beispiel 1

(Vergleichsbeispiel)

Beispiel 1 wurde in der Laboranlage 1 durchgeführt. Über Strom (1a) wurden über Dosierpumpen 1760 g/h Methylformiat und über Strom (1c) 849 g/h Wasser in den Rührkessel A1 zudosiert. Strom (1c) wurde dem Mischbehälter X entnommen, der sich aus Frischwasser via Strom (1b) und Recycle-Wasser aus der Destillationsvorrichtung B2 via Strom (3c) zusammensetzte. Strom (1b) wurde so gewählt, dass die Summe von Strom (1b) und Strom (3c) dem gewünschten Strom (1c) ergab. Der Rührkessel A1 wurde bei 110°C und 1,3 MPa abs betrieben. Der Austrag wurde in Rohrreaktor A2 geleitet, der ebenfalls bei 110°C und 1,3 MPa abs betrieben wurde. Der Austrag von Rohrreaktor A2 wurde in Rohrreaktor A3 geleitet. In diesen wurden 1964 g/h Tri-n-hexylamin über Strom (8a) zugeführt. Der Austrag von Rohrreaktor A3 wurde in Rohrreaktor A4 geleitet. In diesen wurden weitere 1661 g/h Tri-n-hexylamin über Strom (8b) zugeführt. Die Ströme (8a) und (8b) wurden dabei aus dem Behälter Y entnommen, der dazu diente das über Strom (8) rückgeführte Tri-n-hexylamin auf die beiden Rohrreaktoren A3 und A4 zu verteilen. Der Betrieb von Rohrreaktor A3 erfolgte bei 115°C und 1,3 MPa abs, der von Rohrreaktor A4 bei 110°C und 1,3 MPa abs. Als Strom (2) wurde ein Produktgemisch enthaltend 58,4 Gew.-% Tri-n-hexylamin, 16,4 Gew.-% Ameisensäure, 12,3 Gew.-% Methanol, 7,8 Gew.-% Wasser und 6,9 Gew.-% Methylformiat gewonnen.

Strom (2) wurde entspannt und in den Kolonnenkörper der Destillationsvorrichtung B1 geleitet. Bei einem Kopfdruck von 0,18 MPa abs und einem Rücklaufverhältnis von 2,5 wurde als Kopfprodukt Strom (3ab) ein Gemisch enthaltend gebildetes Methanol und nicht umgesetztes Methylformiat abgezogen. Als Sumpfprodukt wurde als Strom (3d) 5012 g/h eines Gemisches enthaltend 71,2 Gew.-% Tri-n-hexylamin, 9,1 Gew.-% Wasser, 20,7 Gew.-% Ameisensäure und 0,1 Gew.-% Methanol erhalten. Die Sumpftemperatur in B1 betrug 117°C.

Strom (3d) wurde in den Kolonnenkörper der Destillationsvorrichtung B2 geleitet. Zusätzlich wurden über Strom (5b) 277 g/h des Seitenabzugs aus dem Kolonnenkörper der Destillationsvorrichtung C1 zugeführt, welcher 79,3 Gew.-% Ameisensäure und 16,6 Gew.-% Wasser enthielt. Als Kopfprodukt der Destillationsvorrichtung B2 wurden bei einem Kopfdruck von 0,10 MPa abs und einem Rücklaufverhältnis von 0,71 450 g/h von Strom (3c) abgezogen. Strom (3c), der 98,8 Gew.-% Wasser und 0,3 Gew.-% Ameisensäure enthielt, wurde dem Mischbehälter X zur Rückführung in den Rührkessel A1 zugeführt.

Über Strom (4) wurden als Sumpfprodukt bei einer Sumpftemperatur in B2 von 160°C 4821 g/h eines Gemisches enthaltend 75,3 Gew.-% Tri-n-hexylamin, 26,0 Gew.-% Ameisensäure und 1,2 Gew.-% Wasser erhalten und von oben auf den Verdampfer C2 geführt. Der Verdampfer C2 und der Kolonnenkörper C1 wurden im Vakuum betrieben. Die Temperatur am unteren Ablauf des Verdampfers C2 betrug 161 °C. Der gasförmige Austrag des Verdampfers wurde als Strom (6x) dem Kolonnenkörper C1 zugeführt.

Dieser wurde bei einem Kopfdruck von 0,015 MPa abs und mit einem Rücklaufverhältnis von Rücklauf zu Destillat von 4 betrieben. Als Kopfprodukt von C1 wurden als Strom (5) 907 g/h 99,6 Gew.-%ige Ameisensäure erhalten. Der n-Hexylformiatgehalt lag bei < 10 Gew.-ppm und der n-Hexanalgehalt bei < 15 Gew.-ppm. Als Seitenabzug wurden 277 g/h als Strom (5b) entnommen und zum Kolonnenkörper B2 rückgeführt. Der flüssige Ablauf des Kolonnenkörpers C1 wurde als Strom (6a) oben in den Verdampfer C2 eingespeist.

Der flüssige Austrag des Verdampfers C2 wurde als Strom (6b) zum Phasentrenngefäß D geleitet. Dieses wurde bei Normaldruck und bei einer Temperatur von 80°C betrieben. Es bildeten sich zwei Flüssigphasen aus. Die obere Flüssigphase wurde kontinuierlich als Strom (8) mit 3587 g/h abgezogen und in den Behälter Y gefördert. Strom (8) enthielt 95,7 Gew.-% Tri-n-hexylamin und 1,2 Gew.-% Ameisensäure. Die untere Flüssigphase wurde kontinuierlich als Strom (7) auf Verdampfer C2 gefahren. Der verbleibende Strom wurde oben in den Verdampfer C2 eingespeist.

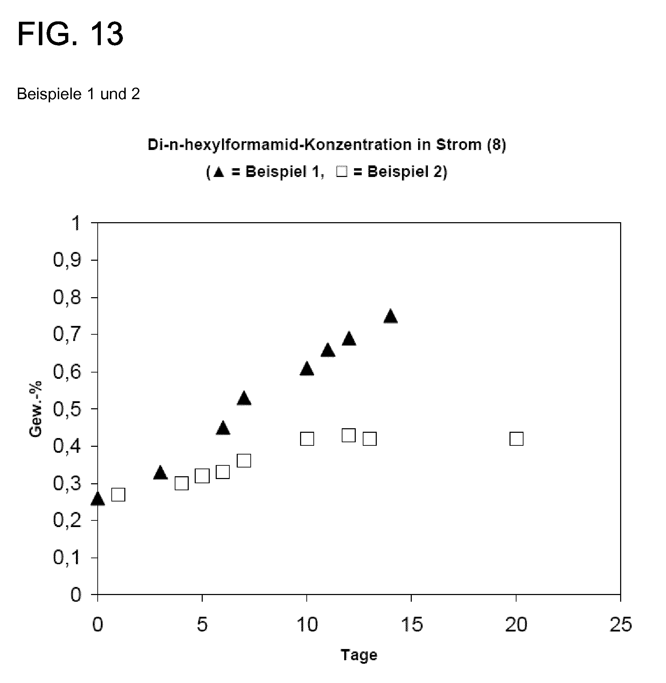

Um den oben genannten Betriebszustand zu gewährleisten, wurde die Anlage zunächst sieben Tage eingefahren. Während dieser Zeit stieg die Di-n-hexylformamid-Konzentration in Strom (8) auf 0,26 Gew.-% an und stieg auch

in den nachfolgenden Tagen stetig weiter. 14 Tage nach dem Einfahren lag die Konzentration bereits bei 0,75 Gew.-%. Ein Ende des Anstiegs war nicht erkennbar. Die Di-n-hexylformamid-Konzentration ist grafisch in Fig. 13 wiedergegeben.

Beispiel 2

(erfindungsgemäßes Beispiel)

Laboranlage 1 wurde nun in Laboranlage 2 umgebaut und dabei um das separate Phasentrenngefäß E erweitert. Die Anlage wurde neu angefahren und innerhalb von sieben Tagen wieder stabile Betriebsparameter erreicht. Bis auf den Bereich um das Phasentrenngefäß E entsprechen diese den in Beispiel 1 genannten Werten.

Im Unterschied zu Beispiel 1 wurde jedoch der als Kopfprodukt der Destillationsvorrichtung B2 abgezogene Strom (3c) von 450 g/h auf das Phasentrenngefäß E geführt, welches bei 30°C betrieben wurde. Strom (3x) wurde daraus von unten entnommen und dem Mischbehälter X zur Rückführung in den Rührkessel A1 zugeführt. Strom (3x) enthielt 99,3 Gew.% Wasser und 0,15 Gew.% Ameisensäure. Einige Tage nach dem Anfahren der Anlage bildete sich langsam eine weitere, obere Phase aus. Diese wurde dann täglich als Strom (3y) entfernt und gesammelt. Nach 13 Tagen (gerechnet ab dem Anfahren der Anlage) wurden insgesamt 2,4 g dieser Oberphase erhalten. Sie enthielt 75,8 Gew.-% Di-n-hexylformamid, 0,3 Gew.-% Tri-n-hexylamin, 0,8 Gew.-% Wasser, 1,2 Gew.-% Ameisensäure, 1,1 Gew.-% n-Hexylformiat, 0,1 Gew.-% n-Hexanol und 0,2 Gew.-% n-Hexanal. Zudem wurden im Gaschromatogramm noch 8,8 Flächen-% C12-Aldolkondensationsprodukte von Hexanal nachgewiesen.

Im Strom (8) betrug die Di-n-hexylformamid-Konzentration acht Tage nach dem Anfahren 0,27 Gew.-%. Im Verlauf der nächsten zehn Tage erhöhte sich die Konzentration zunächst kontinuierlich weiter, erreichte dann aber bei 0,42 Gew.-% einen Sättigungswert.

Beispiel 1 belegt, dass ohne die Anwendung der erfindungsgemäßen Maßnahme zur gezielten Abtrennung und Ausschleusung von organischen Abbauprodukte des tertiären Amins (I), im vorliegenden Beispiel insbesondere von Di-n-hexylformamid, dessen Konzentration in Strom (8) kontinuierlich ansteigt. Beispiel 1 ist zudem auch ein Nachweis dafür, dass sich Di-n-hexylformamid auch unter den realen Betriebsbedingungen bildet. Ernste Probleme beim langfristigen Betrieb eines derartigen Verfahrens wären vorprogrammiert.

Im Gegensatz dazu zeigt Beispiel 2, dass sich Di-n-hexylformamid neben diversen anderen Abbauprodukten des eingesetzten tertiären Amins (I) bereits nach kurzer Betriebszeit von nur wenigen Tagen als Oberphase im Phasentrenngefäß E absetzt und gezielt entfernt werden kann. Die Di-n-hexylformamid-Konzentration in Strom (8) kann dadurch auf einen niedrigen, konstanten Wert gehalten werden. Nachteile wie etwa eine langsam ansteigende Verunreinigung der als Wertprodukt als Strom (5) abgetrennten Ameisensäure sowie einer nachteiligen Beeinflussung des Phasenzerfalls des Sumpfprodukts aus der Destillationsvorrichtung C1/C2, werden somit sicher unterbunden.

Beispiel 3a

(Zersetzung von Tri-n-hexylamin in Gegenwart von Ameisensäure und Wasser)

95,3 g (0,35 Mol) Tri-n-hexylamin, 16,3 g (0,35 Mol) Ameisensäure (98 - 100 Gew.-%) und 6,3 g (0,35 Mol) Wasser wurden im Labor im Eisbad gemischt. Im Anschluss wurde die erhaltene Lösung auf Raumtemperatur (ca. 20°C) erwärmt und durch dreimaliges Anlegen von Vakuum (2 hPa abs) sowie Begasung mit reinem Stickstoff entgast. Es wurde eine zweiphasige Lösung erhalten. Diese wurde dann in einer sogenannten Glovebox unter $N_2$-Atmosphäre in einen 270 mL Autoklaven (Material HC) umgefüllt und verschlossen. Der Autoklav wurde im Anschluss mit Stickstoff auf 1,0 MPa abs aufgepresst und unter starkem Rühren auf 160°C erhitzt. Nach Erreichen der Temperatur wurde mit $N_2$ auf einen Gesamtdruck von 2,5 MPa abs nachgepresst. Die Reaktionsmischung wurde nun 24 Stunden bei 160°C gerührt. Anschließend wurde der Autoklav auf Raumtemperatur abgekühlt, auf Normaldruck entspannt und der Inhalt in ein Glasgefäß überführt. Der Austrag trennte sich in zwei Phasen. Als Oberphase wurden 42,1 g und als Unterphase 68,4 g erhalten. Beide Phasen wurden gaschromatografisch auf ihren Di-n-hexylformamid-Gehalt untersucht. Die obere Phase enthielt 0,10 Gew-%, die untere Phase 0,46 Gew-% Di-n-hexylformamid.

Beispiel 3b

(Zersetzung von Tri-n-hexylamin in Gegenwart von Ameisensäure und Wasser)

95,3 g (0,35 Mol) Tri-n-hexylamin, 16,3 g (0,35 Mol) Ameisensäure (98 - 100 Gew.-%) und 6,3 g (0,35 Mol) Wasser wurden im Labor im Eisbad gemischt. Im Anschluss wurde die erhaltene Lösung auf Raumtemperatur (ca. 20°C) erwärmt und durch dreimaliges Anlegen von Vakuum (2 hPa abs) sowie Begasung mit reinem Stickstoff entgast. Es

wurde eine zweiphasige Lösung erhalten. Diese wurde dann in einer sogenannten Glovebox unter $N_2$-Atmosphäre in einen 270 mL Autoklaven (Material HC) umgefüllt und verschlossen. Der Autoklav wurde im Anschluss mit Stickstoff auf 1,0 MPa abs aufgepresst und unter starkem Rühren auf 160°C erhitzt. Nach Erreichen der Temperatur wurde mit $N_2$ auf einen Gesamtdruck von 2,5 MPa abs nachgepresst. Die Reaktionsmischung wurde nun 72 Stunden bei 160°C gerührt. Anschließend wurde der Autoklav auf Raumtemperatur abgekühlt, auf Normaldruck entspannt und der Inhalt in ein Glasgefäß überführt. Der Austrag trennte sich in zwei Phasen. Als Oberphase wurden 48,1 g und als Unterphase 57,9 g erhalten. Beide Phasen wurden gaschromatografisch auf ihren Di-n-hexylformamid-Gehalt untersucht. Die obere Phase enthielt 0,16 Gew-%, die untere Phase 0,69 Gew-% Di-n-hexylformamid.

Beispiele 3a und 3b zeigen, dass sich Tri-n-hexylamin bei erhöhter Temperatur und unter erhöhtem Druck in Gegenwart von Ameisensäure und Wasser zu Di-n-hexylformamid zersetzt.

Beispiel 4

(Zersetzung von Tri-n-hexylamin in Gegenwart von Sauerstoff)

134,6 g (0,50 Mol) Tri-n-hexylamin und 46,5 g (1,0 Mol) Ameisensäure (98 - 100 Gew.-%) wurden im Labor im Eisbad in einem Rundkolben gemischt. Die erhaltene Lösung wurd dann auf Raumtemperatur (ca. 20°C) erwärmt. Der Rundkolben wurde nun mit einem Rückflusskühler versehen und die Lösung unter Rühren auf 110°C erhitzt. Dabei wurde der Rückflusskühler oben offen gelassen, wodurch die Lösung kontinuierlich mit Luft in Kontakt war. Unter diesen Bedingungen wurde die Lösung 66 Stunden lang gerührt und im Anschluss auf Raumtemperatur abgekühlt. Der Austrag wurde gaschromatografisch analysiert. Es fanden sich darin 1,7 Gew.-% Di-n-hexylformamid und 0,43 Gew.-% n-Hexanal.

Beispiel 4 belegt dass sich Tri-n-hexylamin in ameisensaurer Lösung in Gegenwart von Luftsauerstoff zu Di-n-hexylformamid und n-Hexanal zersetzt.

Beispiele 5 bis 7

(Einfluss von Di-n-hexylformamid auf den Phasenzerfall einer Mischung aus Ameisensäure und Tri-n-hexylamin)

Beispiel 5

In Beispiel 5 wurden in einem mittels Magnetrührer gerührten Glaskolben 243,8 g (0,9 Mol) Tri-n-hexylamin vorgelegt und 41,8 g (0,9 Mol) Ameisensäure (98 - 100 Gew.-%) unter Eisbadkühlung zugetropft. Nach dem Ende der Zugabe wurde die Lösung auf Raumtemperatur (ca. 20°C) erwärmt und anschließend auf 80°C erhitzt und für 30 Minuten bei dieser Temperatur gerührt. Es wurden zwei Phasen erhalten. Von jeder Phase wurde bei 80°C eine Probe genommen und auf den Gehalt an Ameisensäure durch Titration mit 0,1 N NaOH in Isopropanol mit potentiometrischer Endpunktbestimmung analysiert. Der Tri-n-hexylamin-Gehalt wurde jeweils als Rest zu 100% angenommen.

Analytischen Daten (Zusammensetzung bei 80°C):

| | Obere Phase | Untere Phase |
|---|---|---|
| Ameisensäure | 1,0 Gew.-% | 20,5 Gew.-% |
| Tri-n-hexylamin | 99,0 Gew.-% | 79,5 Gew.-% |

Beispiel 6

In Beispiel 6 wurden in einem mittels Magnetrührer gerührten Glaskolben 243,8 g (0,9 Mol) Tri-n-hexylamin und 17,1 g Di-n-hexylformamid vorgelegt und dann 17,1 g (0,37 Mol) Ameisensäure (98 - 100 Gew.-%) unter Eisbadkühlung zugetropft. Nach dem Ende der Zugabe wurde die Lösung auf Raumtemperatur (ca. 20°C) erwärmt und anschließend auf 80°C erhitzt und für 30 Minuten bei dieser Temperatur gerührt. Es wurden ebenfalls zwei Phasen erhalten. Von jeder Phase wurde bei 80°C eine Probe genommen und auf den Gehalt an Ameisensäure durch Titration mit 0,1 N NaOH in Isopropanol mit potentiometrischer Endpunktbestimmung analysiert. Die Gehalte an Di-n-hexylformamid und Tri-n-hexylamin wurden über einen kalibrierten Gaschromatografen ermittelt.

Analytische Daten (Zusammensetzung bei 80°C):

| | Obere Phase | Untere Phase |
|---|---|---|
| Ameisensäure | 2,9 Gew.-% | 15,6 Gew.-% |
| Di-n-hexylformamid | 4,6 Gew.-% | 5,9 Gew.-% |
| Tri-n-hexylamin | 92,1 Gew.-% | 78,0 Gew.-% |

Beispiel 7

In Beispiel 7 wurden in einem mittels Magnetrührer gerührten Glaskolben 243,8 g (0,9 Mol) Tri-n-hexylamin und 28,5 g Di-n-hexylformamid vorgelegt und dann 41,8 g (0,9 Mol) Ameisensäure (98 - 100 Gew.-%) unter Eisbadkühlung zugetropft. Nach dem Ende der Zugabe wurde die Lösung auf Raumtemperatur (ca. 20°C) erwärmt und anschließend auf 80°C erhitzt und für 30 Minuten bei dieser Temperatur gerührt. Im Gegensatz zu den Beispielen 5 und 6 wurde hier nur eine einzige Phase erhalten. Eine Analyse der Zusammensetzung erübrigte sich daher.

Die Beispiele 5, 6 und 7 belegen, dass der Phasenzerfall beim System Tri-n-hexylamin und Ameisensäure durch Di-n-hexylformamid negativ beeinflusst wird. In Beispiel 5 ohne Zugabe von Di-n-hexylformamid enthielt die obere Phase nur 1,0 Gew.-% Ameisensäure. Der Rest war Tri-n-hexylamin. Beispiel 6 zeigt, dass selbst wenn der Phasenzerfall noch aufrecht erhalten bleibt, die Ameisensäure-Konzentration in der oberen Phase durch die phasenvermittelnde Wirkung von Di-n-hexylformamid auf 2,9 Gew.-%, als mehr als den doppelten Wert aus Beispiel 5, steigt, und das obwohl in Beispiel 6 sogar bewusst weniger Ameisensäure zugegeben wurde als in Beispiel 5. Bei einem kontinuierlich betriebenen Verfahren zur Herstellung von Ameisensäure, bei dem der Sumpfaustrag der Ameisensäure-Reinkolonne (siehe Destillationsvorrichtung C1/C2 in Laboranlage 1) in zwei Phasen getrennt und auch getrennt rückgeführt wird, würde eine schlechtere Phasentrennung zwangsläufig die Kreislaufströme unnötig vergrößern. Erhöht man die Di-n-hexylformamid-Menge weiterhin wie in Beispiel 7 gezeigt, so fällt der Phasenzerfall schließlich gänzlich weg.

Beispiele 8 bis 9

(Phasentrennung von Di-n-hexylformamid und Wasser)

Beispiel 8

In Beispiel 8 wurden in einem mittels Magnetrührer gerührten Glaskolben 6,0 g Di-n-hexylformamid und 6,0 g Wasser unter Eisbadkühlung zugetropft. Nach dem Ende der Zugabe wurde die Lösung auf Raumtemperatur (ca. 20°C) erwärmt und anschließend 30 Minuten gerührt. Es wurden zwei Phasen erhalten, welche in einem Phasentrenngefäß getrennt und ausgewogen werden. Von jeder Phase wurden bei 25°C eine Probe genommen. Als Oberphase fielen 5,8 g und als Unterphase 5,5 g an. Der Wassergehalt in beiden Phasen wurde durch Titration nach Karl-Fischer mit potentiometrischer Endpunktbestimmung bestimmt. Der Di-n-hexylformamid-Gehalt wurde jeweils als Rest zu 100% angenommen.

Analytische Daten (Zusammensetzung bei 25°C):

| | Obere Phase | Untere Phase |
|---|---|---|
| Wasser | 2,9 Gew.-% | 99,9 Gew.-% |
| Di-n-hexylformamid | 97,1 Gew.-% | 0,1 Gew.-% |

Beispiel 9

In Beispiel 9 wurden in einem mittels Magnetrührer gerührten Glaskolben 18,4 g Di-n-hexylformamid und 18,4 g Wasser unter Eisbadkühlung zugetropft. Nach dem Ende der Zugabe wurde die Lösung zunächst auf Raumtemperatur (ca. 20°C) und anschließend weiter auf 100°C erwärmt und bei 100°C für 30 Minuten unter Rückfluss gerührt. Auch bei 100°C wurden zwei Phasen erhalten. Der Wassergehalt in beiden Phasen wurde ebenfalls durch Titration nach Karl-Fischer mit potentiometrischer Endpunktbestimmung bestimmt. Der Di-n-hexylformamid-Gehalt wurde jeweils als Rest zu 100% angenommen.

Analytische Daten (Zusammensetzung bei 100°C):

| | Obere Phase | Untere Phase |
|---|---|---|
| Wasser | 4,3 Gew.% | 99,9 Gew.% |
| Di-n-hexylformamid | 95,7 Gew.% | 0,1 Gew.% |

Beispiel 8 und 9 belegen, dass das System Di-n-hexylformamid und Wasser sowohl bei Raumtemperatur als auch bei erhöhter Temperatur eine Mischungslücke aufweist.

Beispiel 10

(Zersetzung von Tri-n-pentylamin in Gegenwart von Ameisensäure und Wasser)

81,2 g (0,35 Mol) Tri-n-pentylamin, 16,27 g (0,35 Mol) Ameisensäure (98 - 100 Gew.-%) und 6,31 g Wasser wurden im Labor im Eisbad gemischt. Im Anschluss wurde die erhaltene Lösung auf Raumtemperatur (ca. 20°C) erwärmt und durch dreimaliges Anlegen von Vakuum (2 hPa abs) sowie Begasung mit reinem Stickstoff entgast. Es wurde eine zweiphasige Lösung erhalten. Diese wurde dann in einer sogenannten Glovebox unter $N_2$-Atmosphäre in einen 270 mL Autoklaven (Material HC) umgefüllt und verschlossen. Der Autoklav wurde im Anschluss mit Stickstoff auf 1,0 MPa aufgepresst und unter starkem Rühren auf 160°C erhitzt. Nach Erreichen der Temperatur wurde mit $N_2$ auf einen Gesamtdruck von 2,5 MPa abs nachgepresst Die Reaktionsmischung wurde nun 24 Stunden bei 160°C gerührt. Anschließend wurde der Autoklav auf Raumtemperatur abgekühlt, auf Normaldruck entspannt und der Inhalt in ein Glasgefäß überführt. Der Austrag trennte sich in zwei Phasen. Als Oberphase wurden 36,8 g und als Unterphase 59,3 g erhalten. Beide Phasen wurden gaschromatografisch auf ihren Di-n-pentylformamid-Gehalt untersucht. Die obere Phase enthielt 0,15 Gew.%, die untere Phase 0,45 Gew.%.

Beispiel 11

(Zersetzung von Tri-n-octylamin in Gegenwart von Ameisensäure und Wasser)

123,6 g (0,35 Mol) Tri-n-octylamin, 16,3 g (0,35 Mol) Ameisensäure (98 - 100 Gew.-%) und 6,3 g Wasser wurden im Labor im Eisbad gemischt. Im Anschluss wurde die erhaltene Lösung auf Raumtemperatur (ca. 20°C) erwärmt und durch dreimaliges Anlegen von Vakuum (2 hPa abs) sowie Begasung mit reinem Stickstoff entgast. Es wurde eine zweiphasige Lösung erhalten. Diese wurde dann in einer sogenannten Glovebox unter $N_2$-Atmosphäre in einen 270 mL Autoklaven (Material HC) umgefüllt und verschlossen. Der Autoklav wurde im Anschluss mit Stickstoff auf 1,0 MPa aufgepresst und unter starkem Rühren auf 160°C erhitzt. Nach Erreichen der Temperatur wurde mit $N_2$ auf einen Gesamtdruck von 2,5 MPa abs nachgepresst. Die Reaktionsmischung wurde nun 24 Stunden bei 160°C gerührt. Anschließend wurde der Autoklav auf Raumtemperatur abgekühlt, auf Normaldruck entspannt und der Inhalt in ein Glasgefäß überführt. Der Austrag trennte sich in zwei Phasen. Als organische Oberphase wurden 138,2 g und eine wässrige Unterphase von 1,3 g erhalten. Beide Phasen wurden gaschromatografisch auf ihren Di-n-octylformamid-Gehalt untersucht. Die obere Phase enthielt 0,32 Gew-%, die untere Phase < 0,1 Gew.-%.

## Patentansprüche

**1.** Verfahren zur Gewinnung von Ameisensäure durch thermische Trennung eines Stroms enthaltend Ameisensäure und ein tertiäres Amin (I), welches bei einem Druck von 1013 hPa abs einen um mindestens 5°C höheren Siedepunkt als Ameisensäure aufweist und welches die allgemeine Formel (I)

$$NR^1R^2R^3 \qquad (I)$$

aufweist, in der die Reste $R^1$ bis $R^3$ unabhängig voneinander ausgewählt sind aus der Gruppe $C_5$- bis $C_8$-Alkyl, bei dem man

(a) durch Zusammenbringen von tertiärem Amin (I) und einer Ameisensäurequelle in Gegenwart von Wasser einen flüssigen Strom enthaltend Ameisensäure, tertiäres Amin (I) und Wasser erzeugt, wobei

(i) man eine Ameisensäurequelle einsetzt, welche Methylformiat enthält und aus welcher der flüssige Strom enthaltend Ameisensäure, tertiäres Amin (I) und Wasser durch Hydrolyse von Methylformiat erhalten wird;

oder

(ii) man eine Ameisensäurequelle einsetzt, welche Kohlendioxid enthält und aus welcher der flüssige Strom enthaltend Ameisensäure, tertiäres Amin (I) und Wasser durch übergangsmetallkatalysierte Hydrierung von Kohlendioxid erhalten wird,

und der flüssige Strom enthaltend Ameisensäure, tertiäres Amin (I) und Wasser ein Molverhältnis von Ameisensäure zu tertiärem Amin (I) von 0,5 bis 5 aufweist;

(b) aus dem aus Schritt (a) erhaltenen flüssigen Strom Wasser und organische Abbauprodukte des tertiären Amins (I) abtrennt, wobei die organischen Abbauprodukte des tertiären Amins (I) bereits in dem Schritt (a) zugeführten tertiären Amin (I) enthalten waren und/oder im Laufe des Verfahrens bis zum vorliegenden Schritt (b) entstanden, und einen an Wasser und organischen Abbauprodukten des tertiären Amins (I) abgereicherten flüssigen Strom enthaltend Ameisensäure und tertiäres Amin (I) erhält; und

(c) aus dem aus Schritt (b) erhaltenen flüssigen Strom enthaltend Ameisensäure und tertiäres Amin (I) in einer Destillationsvorrichtung bei einer Sumpftemperatur von 100 bis 300°C und einem Druck von 30 bis 3000 hPa abs Ameisensäure destillativ entfernt;

**dadurch gekennzeichnet, dass** man

(b1) den in Schritt (b) abgetrennten Strom enthaltend Wasser und organische Abbauprodukte des tertiären Amins (I) in zwei flüssige Phasen trennt;

(b2) die obere, an organischen Abbauprodukten des tertiärem Amins (I) angereicherte Flüssighase entfernt; und

(b3) die untere, Wasser enthaltende Flüssigphase zu Schritt (a) rückführt.

2. Verfahren nach Anspruch 1, **dadurch gekennzeichnet, dass** man die Abtrennung in Schritt (b) destillativ durchführt.

3. Verfahren nach den Ansprüchen 1 bis 2, **dadurch gekennzeichnet, dass** man

(i) in Schritt (a) eine Ameisensäurequelle einsetzt, welche Methylformiat enthält und aus welcher ein flüssiger Strom enthaltend Ameisensäure, tertiäres Amin (I), Wasser und Methanol durch Hydrolyse von Methylformiat erhalten wird; und

(ii) in Schritt (b) aus dem aus Schritt (a) erhaltenen Strom einen weiteren Strom enthaltend das aus der Spaltung von Methylformiat entstandene Methanol abtrennt.

4. Verfahren nach Anspruch 3, **dadurch gekennzeichnet, dass** man

(i) in Schritt (b) aus dem aus Schritt (a) erhaltenen Strom einen weiteren Strom enthaltend nicht umgesetztes Methylformiat abtrennt; und

(ii) das abgetrennte Methylformiat zu Schritt (a) rückführt.

5. Verfahren nach den Ansprüchen 1 bis 2, **dadurch gekennzeichnet, dass** man

(i) in Schritt (a) in Gegenwart von Methanol eine Ameisensäurequelle einsetzt, welche Kohlendioxid, Wasserstoff und einen homogenen Katalysator enthält und aus welcher ein flüssiger Strom enthaltend Ameisensäure, tertiäres Amin (I), Wasser und Methanol durch homogen-katalysierte Hydrierung von Kohlendioxid erhalten wird; und

(ii) in Schritt (b) aus dem aus Schritt (a) erhaltenen Strom einen weiteren Strom enthaltend Methanol abtrennt und das abgetrennte Methanol zu Schritt (a) rückführt.

6. Verfahren nach den Ansprüchen 1 bis 5, **dadurch gekennzeichnet, dass** man das in Schritt (a) einzusetzende tertiäre Amin (I) und die Trennrate in der in Schritt (c) genannten Destillationsvorrichtung so wählt, dass sich im Sumpfaustrag der in Schritt (c) genannten Destillationsvorrichtung zwei flüssige Phasen bilden,

(d) den Sumpfaustrag aus der in Schritt (c) genannten Destillationsvorrichtung in zwei flüssige Phasen trennt, wobei die obere Flüssigphase ein Molverhältnis von Ameisensäure zu tertiärem Amin (I) von 0 bis 0,5 und die untere Flüssigphase ein Molverhältnis von Ameisensäure zu tertiärem Amin (I) von 0,5 bis 4 aufweist;

(e) die obere Flüssighase der Phasentrennung aus Schritt (d) zu Schritt (a) rückführt; und

(f) die untere Flüssigphase der Phasentrennung aus Schritt (d) zu Schritt (b) und/oder (c) rückführt.

7. Verfahren nach Anspruch 6, **dadurch gekennzeichnet, dass** man in Schritt (e) 10 bis 100% der oberen Flüssighase der Phasentrennung aus Schritt (d) zu Schritt (a) rückführt.

**8.** Verfahren nach Anspruch 6, **dadurch gekennzeichnet, dass** man in Schritt (e) 90 bis 100% der oberen Flüssighase der Phasentrennung aus Schritt (d) zu Schritt (a) rückführt.

**9.** Verfahren nach den Ansprüchen 1 bis 8, **dadurch gekennzeichnet, dass** der in Schritt (a) erzeugte flüssige Strom eine Konzentration an Ameisensäure plus tertiärem Amin (I) von 1 bis 99 Gew.-% bezogen auf die Gesamtmenge des Stroms aufweist.

**10.** Verfahren nach den Ansprüchen 1 bis 9, **dadurch gekennzeichnet, dass** man die Trennrate in der in Schritt (c) genannten Destillationsvorrichtung so wählt, dass das Molverhältnis von Ameisensäure zu tertiärem Amin (I) im Sumpfaustrag 0,1 bis 2,0 beträgt.

**11.** Verfahren nach den Ansprüchen 1 bis 10, **dadurch gekennzeichnet, dass** man als tertiäres Amin (I) Tri-n-pentylamin, Tri-n-hexylamin, Tri-n-heptylamin oder Tri-n-octylamin einsetzt.

## Claims

**1.** A process for obtaining formic acid by thermal separation of a stream comprising formic acid and a tertiary amine (I) which at a pressure of 1013 hPa abs has a boiling point which is at least 5°C higher than that of formic acid and which has the general formula (I)

$$NR^1R^2R^3 \qquad (I)$$

in which the radicals $R^1$ to $R^3$ are selected independently from the group consisting of $C_5$-$C_8$-alkyl, in which

(a) a liquid stream comprising formic acid, tertiary amine (I) and water is produced by combining tertiary amine (I) and a formic acid source in the presence of water, where

(i) a formic acid source comprising methyl formate is used and the liquid stream comprising formic acid, tertiary amine (I) and water is obtained therefrom by hydrolysis of methyl formate; or
(ii)a formic acid source which comprises carbon dioxide is used and the liquid stream comprising formic acid, tertiary amine (I) and water is obtained therefrom by transition metal-catalyzed hydrogenation of carbon dioxide,
and the liquid stream comprising formic acid, tertiary amine (I) and water has a molar ratio of formic acid to tertiary amine (I) of from 0.5 to 5;

(b) water and organic decomposition products of the tertiary amine (I) are separated off from the liquid stream obtained from step (a), with the organic decomposition products of the tertiary amine (I) having been comprised in the tertiary amine (I) fed to step (a) and/or been formed during the course of the process up to the present step (b), and a liquid stream which comprises formic acid and tertiary amine (I) and is depleted in water and organic decomposition products of the tertiary amine (I) is obtained; and
(c) formic acid is removed by distillation from the liquid stream comprising formic acid and tertiary amine (I) obtained from step (b) in a distillation apparatus at a temperature at the bottom of from 100 to 300°C and a pressure of from 30 to 3000 hPa abs;
wherein
(b1) the stream comprising water and organic decomposition products of the tertiary amine (I) which has been separated off in step (b) is separated into two liquid phases;
(b2) the upper liquid phase enriched in organic decomposition products of the tertiary amine (I) is removed; and
(b3) the lower, water-comprising liquid phase is recirculated to step (a).

**2.** The process according to claim 1, wherein the separation in step (b) is carried out by distillation.

**3.** The process according to claim 1 or 2, wherein

(i) a formic acid source comprising methyl formate is used and a liquid stream comprising formic acid, tertiary amine (I), water and methanol is obtained therefrom by hydrolysis of methyl formate in step (a); and
(ii) a further stream comprising the methanol formed by dissociation of methyl formate is separated off from the stream obtained from step (a) in step (b).

**4.** The process according to claim 3, wherein

(i) a further stream comprising unreacted methyl formate is separated off from the stream obtained from step (a) in step (b); and
(ii) the methyl formate which has been separated off is recirculated to step (a).

**5.** The process according to claim 1 or 2, wherein

(i) a formic acid source which comprises carbon dioxide, hydrogen and a homogeneous catalyst is used in the presence of methanol in step (a) and a liquid stream comprising formic acid, tertiary amine (I), water and methanol is obtained therefrom by homogeneously catalyzed hydrogenation of carbon dioxide; and
(ii) a further stream comprising methanol is separated off from the stream obtained from step (a) in step (b) and the methanol which has been separated off is recirculated to step (a).

**6.** The process according to any of claims 1 to 5, wherein the tertiary amine (I) to be used in step (a) and the degree of separation in the distillation apparatus mentioned in step (c) are selected so that two liquid phases are formed in the bottom output from the distillation apparatus mentioned in step (c),

(d) the bottom output from the distillation apparatus mentioned in step (c) is separated into two liquid phases, where the upper liquid phase has a molar ratio of formic acid to tertiary amine (I) of from 0 to 0.5 and the lower liquid phase has a molar ratio of formic acid to tertiary amine (I) of from 0.5 to 4;
(e) the upper liquid phase from the phase separation in step (d) is recirculated to step (a); and
(f) the lower liquid phase from the phase separation in step (d) is recirculated to step (b) and/or (c).

**7.** The process according to claim 6, wherein in step (e) from 10 to 100% of the upper liquid phase from the phase separation in step (d) is recirculated to step (a).

**8.** The process according to claim 6, wherein in step (e) from 90 to 100% of the upper liquid phase from the phase separation in step (d) is recirculated to step (a).

**9.** The process according to any of claims 1 to 8, wherein the liquid stream produced in step (a) has a concentration of formic acid plus tertiary amine (I) of from 1 to 99% by weight, based on the total amount of the stream.

**10.** The process according to any of claims 1 to 9, wherein the degree of separation in the distillation apparatus mentioned in step (c) is selected so that the molar ratio of formic acid to tertiary amine (I) in the bottom output is from 0.1 to 2.0.

**11.** The process according to any of claims 1 to 10, wherein tri-n-pentyl amine, tri-n-hexyl amine, tri-n-heptyl amine or tri-n-octyl amine is used as tertiary amine (I).

**Revendications**

**1.** Procédé d'obtention d'acide formique par séparation thermique d'un courant contenant de l'acide formique et une amine tertiaire (I), qui présente à une pression de 1 013 hPa abs un point d'ébullition supérieur d'au moins 5 °C à l'acide formique et qui présente la formule générale (I)

$$NR^1R^2R^3 \qquad (I)$$

dans laquelle les radicaux $R^1$ à $R^3$ sont choisis indépendamment les uns des autres dans le groupe constitué par les alkyles en $C_5$ à $C_8$, selon lequel

(a) un courant liquide contenant de l'acide formique, l'amine tertiaire (I) et de l'eau est formé par combinaison de l'amine tertiaire (I) et d'une source d'acide formique en présence d'eau,

(i) une source d'acide formique qui contient du formiate de méthyle et à partir de laquelle le courant liquide contenant de l'acide formique, l'amine tertiaire (I) et de l'eau est obtenu par hydrolyse du formiate de méthyle étant utilisée ;
ou

(ii) une source d'acide formique qui contient du dioxyde de carbone et à partir de laquelle le courant liquide contenant de l'acide formique, l'amine tertiaire (I) et de l'eau est obtenu par hydrogénation catalysée par un métal de transition du dioxyde de carbone étant utilisée, et le courant liquide contenant de l'acide formique, l'amine tertiaire (I) et de l'eau présentant un rapport molaire entre l'acide formique et l'amine tertiaire (I) de 0,5 à 5 ;

(b) l'eau et les produits de décomposition organiques de l'amine tertiaire (I) sont séparés du courant liquide obtenu à l'étape (a), les produits de décomposition organiques de l'amine tertiaire (I) étant déjà contenus dans l'amine tertiaire (I) introduite dans l'étape (a) et/ou formés au cours du procédé jusqu'à la présente étape (b), et un courant liquide appauvri en eau et en produits de décomposition organiques de l'amine tertiaire (I), contenant de l'acide formique et l'amine tertiaire (I), est obtenu ;
et
(c) l'acide formique est séparé par distillation du courant liquide contenant de l'acide formique et l'amine tertiaire (I) obtenu à l'étape (b) dans un dispositif de distillation à une température de fond de 100 à 300 °C et à une pression de 30 à 3 000 hPa abs ;
**caractérisé en ce que**
(b1) le courant contenant de l'eau et des produits de décomposition organiques de l'amine tertiaire (I) séparé à l'étape (b) est séparé en deux phases liquides ;
(b2) la phase liquide supérieure, enrichie en produits de décomposition organiques de l'amine tertiaire (I), est séparée ; et
(b3) la phase liquide inférieure, contenant de l'eau, est recyclée dans l'étape (a).

**2.** Procédé selon la revendication 1, **caractérisé en ce que** la séparation à l'étape (b) est réalisée par distillation.

**3.** Procédé selon les revendications 1 à 2, **caractérisé en ce que**

(i) à l'étape (a), une source d'acide formique qui contient du formiate de méthyle et à partir de laquelle un courant liquide contenant de l'acide formique, l'amine tertiaire (I), de l'eau et du méthanol est obtenu par hydrolyse du formiate de méthyle est utilisée ; et
(ii) à l'étape (b), un courant supplémentaire contenant le méthanol formé lors du clivage du formiate de méthyle est séparé à partir du courant obtenu à l'étape (a).

**4.** Procédé selon la revendication 3, **caractérisé en ce que**

(i) à l'étape (b), un courant supplémentaire contenant du formiate de méthyle non réagi est séparé à partir du courant obtenu à l'étape (a) ; et
(ii) le formiate de méthyle séparé est recyclé dans l'étape (a).

**5.** Procédé selon les revendications 1 à 2, **caractérisé en ce que**

(i) à l'étape (a), une source d'acide formique qui contient du dioxyde de carbone, de l'hydrogène et un catalyseur homogène et à partir de laquelle un courant liquide contenant de l'acide formique, l'amine tertiaire (I), de l'eau et du méthanol est obtenu par hydrogénation sous catalyse homogène du dioxyde de carbone est utilisée en présence de méthanol ; et
(ii) à l'étape (b), un courant supplémentaire contenant du méthanol est séparé à partir du courant obtenu à l'étape (a) et le méthanol séparé est recyclé dans l'étape (a).

**6.** Procédé selon les revendications 1 à 5, **caractérisé en ce que** l'amine tertiaire (I) utilisée à l'étape (a) et le taux de séparation dans le dispositif de distillation mentionné à l'étape (c) sont choisis de sorte que deux phases liquides se forment dans la sortie de fond du dispositif de distillation mentionné à l'étape (c),

(d) la sortie de fond du dispositif de distillation mentionné à l'étape (c) est séparée en deux phases liquides, la phase liquide supérieure présentant un rapport molaire entre l'acide formique et l'amine tertiaire (I) de 0 à 0,5 et la phase liquide inférieure présentant un rapport molaire entre l'acide formique et l'amine tertiaire (I) de 0,5 à 4 ;
(e) la phase liquide supérieure de la séparation de phases de l'étape (d) est recyclée dans l'étape (a) ; et
(f) la phase liquide inférieure de la séparation de phases de l'étape (d) est recyclée dans l'étape (b) et/ou (c).

**7.** Procédé selon la revendication 6, **caractérisé en ce qu'**à l'étape (e), 10 à 100 % de la phase liquide supérieure

de la séparation de phases de l'étape (d) est recyclée dans l'étape (a).

8. Procédé selon la revendication 6, **caractérisé en ce qu'**à l'étape (e), 90 à 100 % de la phase liquide supérieure de la séparation de phases de l'étape (d) est recyclée dans l'étape (a).

9. Procédé selon les revendications 1 à 8, **caractérisé en ce que** le courant liquide formé à l'étape (a) présente une concentration en acide formique plus amine tertiaire (I) de 1 à 99 % en poids, par rapport à la quantité totale du courant.

10. Procédé selon les revendications 1 à 9, **caractérisé en ce que** le taux de séparation dans le dispositif de distillation mentionné à l'étape (c) est choisi de sorte que le rapport molaire entre l'acide formique et l'amine tertiaire (I) dans la sortie de fond soit de 0,1 à 2,0.

11. Procédé selon les revendications 1 à 10, **caractérisé en ce que** la tri-n-pentylamine, la tri-n-hexylamine, la tri-n-heptylamine ou la tri-n-octylamine est utilisée en tant qu'amine tertiaire (I).

FIG. 1

E
3y
3
3x
5
1
A
2
B
4
C
8
6

FIG. 2

E
3y
3x
3c
3a/b
5
1
A
2
B
4
C
8
6

FIG. 3

E
3y
3
3x
5
1
A
2
B
4
C
6
7
D
8

FIG. 4

E
3y
3x
3c
3a/b
5
1
A
2
B
4
C
6
7
D
8

FIG. 5

3b
5
5a
3a
1a
5b
1b
A
B
C
4
2
3c
7
6
E
D
3y
3x
8

FIG. 6

3b
1a
3a
5
5a
5b
1b
A
B
C
4
2
3c
7
6
E
D
3y
3x
8

# FIG. 7

3b
1a
3a
5
5a
5b
A
B
C
2
4
1b
3c
7
6
E
D
3y
3x
8

FIG. 8

(a)
3b
1a
3a
2
1b
3c
4
(b)
3b
1a
2
3a
1b
3c
4
(c)
3b
3c
1a
3a
2
1b
4
(d)
3b
1a
3a
2
1b
4
3c
(e)
3b
3c
1a
3a
2
1b
4

FIG.9

(a)
3b
3a
3c
1a
2
1b
4

(b)
3b
3a
1a
2
4
3c

(c)
3b
3c
1a
2
1b
4
3a

FIG. 10

5
5a
5b
C
6
D
7
4
3a
3c
B
E
3y
2
3b
A3
2c
A2
2d
2b
2a
A1
A
1a
1b
8
3x

# FIG. 11

Beispiel 1

5
C1
D
6x
6a
6b
7
C2
4
7
6a
4
B2
5b
3d
3d
B1
3a/b
8
3c
2
A4
8b
A3
Y
8a
A2
X
1b
1c
A1
1a

# FIG. 12

Beispiel 2

5
C1
D
6x
6a
6b
7
C2
4
7
6a
3c
B2
4
E
3y
3d
3d
B1
3a/b
5b
8
2
3x
A4
8b
A3
Y
A2
8a
X
1b
1c
A1
1a

# FIG. 13

Beispiele 1 und 2

Di-n-hexylformamid-Konzentration in Strom (8)
(▲ = Beispiel 1, □ = Beispiel 2)

Gew.-%
1
0,9
0,8
0,7
0,6
0,5
0,4
0,3
0,2
0,1
0
0
5
10
15
20
25
Tage

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 61577703 B
- DE 2545658 A1
- WO 2006021411 A
- EP 0001432 A
- DE 3428319 A
- EP 0181078 A
- EP 0126524 A
- WO 2008116799 A
- EP 0563831 A
- EP 2011060770 W
- DE 102009046310 A1
- EP 2011060012 W
- WO 2012000799 A
- EP Z2011060770 W


### In der Beschreibung aufgeführte Nicht-Patentliteratur


- **C. REICHARDT.** Solvents and Solvent Effects in Organic Chemistry. Wiley-VCH Verlag GmbH & Co KGaA, 2003, 67